(19) **Europäisches Patentamt / European Patent Office / Office européen des brevets**

(11) **EP 4 578 947 A1**

(12) **EUROPEAN PATENT APPLICATION**
published in accordance with Art. 153(4) EPC

(43) Date of publication:
**02.07.2025 Bulletin 2025/27**

(21) Application number: **23857003.0**

(22) Date of filing: **07.07.2023**

(51) International Patent Classification (IPC):
*C12N 15/11* [(2006.01)]  *A01K 67/027* [(2024.01)]
*A61K 35/12* [(2015.01)]  *A61K 48/00* [(2006.01)]
*A61P 43/00* [(2006.01)]  *C12N 1/15* [(2006.01)]
*C12N 1/19* [(2006.01)]  *C12N 1/21* [(2006.01)]
*C12N 5/10* [(2006.01)]  *C12N 15/52* [(2006.01)]

(52) Cooperative Patent Classification (CPC):
A01K 67/027; A61K 35/12; A61K 48/00;
A61P 43/00; C12N 5/10; C12N 15/11; C12N 15/52;
C12N 15/74; C12N 15/80; C12N 15/81

(86) International application number:
**PCT/JP2023/025274**

(87) International publication number:
**WO 2024/042886 (29.02.2024 Gazette 2024/09)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **26.08.2022 JP 2022135305**

(71) Applicant: **OSAKA UNIVERSITY**
**Suita-shi**
**Osaka 565-0871 (JP)**

(72) Inventor: **YAMAMOTO Masahiro**
**Suita-shi, Osaka 565-0871 (JP)**

(74) Representative: **J A Kemp LLP**
**80 Turnmill Street**
**London EC1M 5QU (GB)**

(54) **NON-HUMAN ORGANISM CAPABLE OF SPECIFICALLY ELIMINATING SPECIFIED CELLS**

(57)    The present invention relates to a combination of DNA constructs comprising a first DNA construct containing a first site-specific recombinase gene operably positioned relative to a promoter of a first gene characteristic of specific cells, up to an n-th DNA construct containing an n-th site-specific recombinase gene operably positioned relative to a promoter of an n-th gene characteristic of the cells, and a DNA construct containing a killing gene operably positioned relative to a constitutive promoter, wherein transcription termination sequences flanked between sequences recognized and excised by first to n-th site-specific recombinases are positioned between the constitutive promoter and the killing gene; cells into which the combination has been introduced; and non-human organisms containing the cells.

EP 4 578 947 A1

**Description**

[Technical Field]

The present invention relates to a non-human organism capable of specifically removing specific cells. More specifically, it relates to cells that can be specifically removed and non-human organisms containing such cells, as well as systems (DNA constructs) for producing them. The present invention also relates to methods for analyzing the functions of specific cells using the non-human organism. Furthermore, the present invention relates to pharmaceutical compositions containing the cells.

[Background Art]

Immune cells have various cell lineages including B cells, T cells, macrophages, dendritic cells, natural killer (NK) cells, and others. In analyzing such diverse immune cells, it is important to specifically label and detect each cell type. Furthermore, it becomes possible to infer the functions of these cells based on phenotypes lost when each cell type is removed. Therefore, it is extremely useful to be able to specifically kill each cell type.

A method currently in widespread use for specifically killing and furthermore labeling (targeting) each cell lineage involves crossing transgenic mice expressing Cre recombinase under the promoter of genes specific to each cell subset with mice having YFP (a gene for labeling cells (labeling gene)) or diphtheria toxin receptor (DTR, a gene for killing cells (killing gene)) inserted at the ROSA26 gene locus with a loxP-flanked polyA addition signal sequence (loxP-stop-loxP = LSL sequence) on the 5' side under a constitutive promoter (ROSA26-LSL-YFP mice or ROSA26-LSL-DTR mice) (Non Patent Literature 1). Through this method, B cells can be labeled in CD19-Cre/ROSA26-LSL-YFP mice and removed in CD19-Cre/ROSA26LSL-DTR mice. Furthermore, when analyzing T cells, macrophages, dendritic cells, or NK cells, CD4-Cre, Cx3cr1-Cre, CD11c-Cre, or NKp46-Cre mice are respectively crossed with ROSA26-LSL-YFP·DTR mice, and these are widely used in immunology.

In recent years, it has become clear that these cell lineages have various subsets. For example, CD4-positive T cells have subsets such as Th1, Th2, Th17, regulatory T (Treg), Tfh, etc., and they express specific transcription factors such as Tbet, Gata3, RORgt, Foxp3, Bcl6, respectively.

However, targeting of such CD4-positive T cell subsets, except for Treg cells which can be specifically labeled by Foxp3, could not be analyzed using the above ROSA26-LSL-YFP·DTR mice because Tbet, Gata3, RORgt, and Bcl6 are also expressed in cells other than T cells.

Moreover, even regarding Treg cells that specifically express Foxp3, subsets of Treg cells (i.e., subsets of CD4-positive cell subsets) have recently been discovered (Non Patent Literatures 2-7). Such Th1-Treg, Th2-Treg, Th17-Treg, and Tfh-Treg cells that express Tbet, Gata3, RORgt, and Bcl6 in addition to Foxp3 could not be targeted for the same reason. Furthermore, this was similarly true for B cells, macrophages, dendritic cells, and NK cells, and functional analysis of their immune cell subsets has not been accomplished.

Regarding the analysis of such cell subsets, attempts have been made to analyze various cell subpopulations in the brain using intersectional genetic methods employing Cre/loxP or Flp/FRT dual recombination systems or binary split Cre recombination systems (Non Patent Literatures 8 and 9). However, even when using such systems, specific cell subsets could not be removed, and targeting has not been achieved.

[Citation List]

[Non Patent Literature]

[NPL 1] Buch, T., et al., Nat Methods, 2005, 2, 419-4 26.
[NPL 2] Koch, M.A., et al., Immunity, 2012, 37, 501-510.
[NPL 3] Koch, M.A., et al., Nat Immunol, 2009, 10, 595-602.
[NPL 4] Chung, Y., et al., Nat Med, 2011, 17, 983-988.
[NPL 5] Duhen, T., et al., Blood, 2012, 119, 4430-4440.
[NPL 6] Linterman, M.A., et al., Nat Med, 2011, 17, 975-982.
[NPL 7] Zheng, Y., et al., Nature, 2009, 458, 351-356.
[NPL 8] Kim, J.S., et al., Immunity, 2021, 54, 176-190 e177.
[NPL 9] Madisen, L., et al., Neuron, 2015, 85, 942-958.

[Summary of Invention]

[Technical Problem]

The present invention was made in view of the problems in the conventional technology described above, and aims to provide a non-human organism capable of specifically removing specific cells and a system for producing such organism.

[Solution to Problem]

Many immune cell subsets can be labeled by combining two characteristic markers. For example, Th1-Treg cells are cells that express two characteristic genes, Foxp3 and Tbet. Therefore, to achieve the above objective, we attempted to develop a non-human organism that could express killing genes such as DTR only when these characteristic multiple gene expressions occur, considering that this would be sufficient.

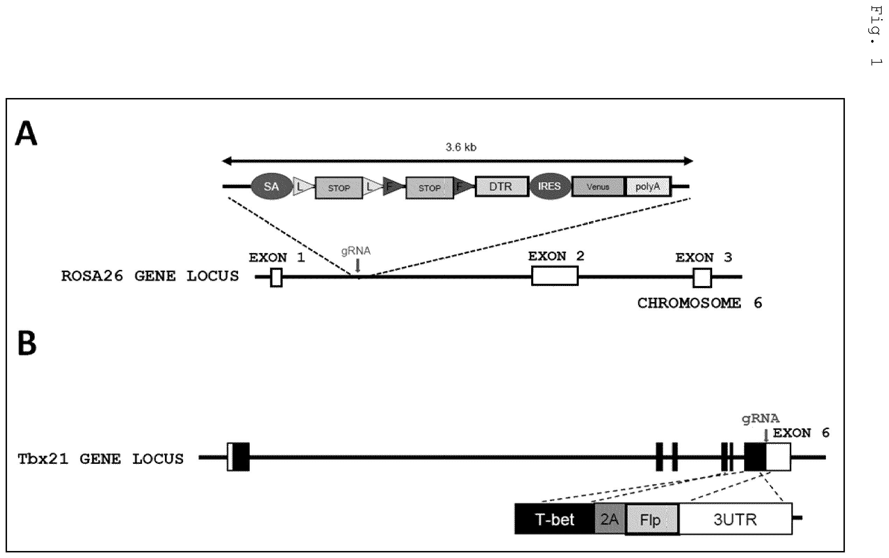

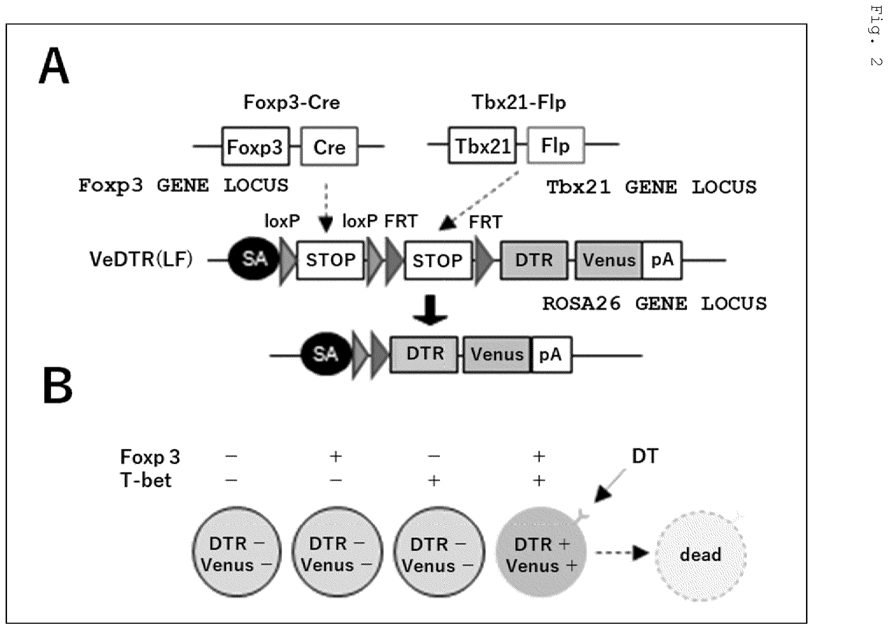

First, we conceived of utilizing the Flp-FRT system in addition to the Cre-loxP system. More specifically, since the Flp-FRT system, analogous to the Cre-loxP system, can delete regions flanked by two FRT sequences using Flp recombinase, we envisioned a gene locus where an FRT-flanked polyA addition signal (FRT-stop-FRT = FSF sequence) was placed in tandem with the LSL sequence upstream of DTR (killing gene) and Venus (labeling gene) (Fig. 1A). We hypothesized that with such a gene locus, DTR and Venus could not be expressed when either Cre or Flp alone was expressed, but their expression would be possible only when both Cre and Flp were expressed (Fig. 2A and B).

To demonstrate this concept, we created mice with LSL-FSF sequences inserted at the ROSA26 gene locus (ROSA26-LSL-FSF-Venus·DTR mice, VeDTR mice).

Next, we examined whether VeDTR mice could specifically label Th1-Treg cells. While we used Foxp3-Cre mice, which are commonly used for Cre expression specific to Treg cells, since there were no mice expressing Flp in Tbet-expressing cells, we created knock-in mice with Flp recombinase inserted at the Tbx21 gene locus encoding Tbet (Tbx21-Flp mice) and produced descendant mice by successively crossing to create Foxp3-Cre/Tbx21-Flp/VeDTR mice.

To examine whether Foxp3-Cre/Tbx21-Flp/VeDTR mice could specifically label Th1-Treg cells, we cultured naive CD4-positive T cells (Th0 cells) derived from the spleens of four types of mice - Foxp3-Cre/Tbx21-Flp/VeDTR mice, Foxp3-Cre/VeDTR (ΔFRT) mice, Tbx21-Flp/VeDTR (ΔloxP) mice, and VeDTR mice - under Th1 differentiation conditions, Treg differentiation conditions, and furthermore Th1-Treg differentiation conditions, and examined Venus expression by flow cytometry (Example 2).

As a result, under Th1 differentiation conditions, Venus-positive cells were observed in Tbx21-Flp/VeDTR (ΔloxP) mice, but Venus expression was not observed in Foxp3-Cre/Tbx21-Flp/VeDTR mice, Foxp3-Cre/VeDTR (ΔFRT) mice, and VeDTR mice. Additionally, under Treg differentiation conditions, Venus expression was observed in Foxp3-Cre/VeDTR (ΔFRT) mice, but Venus expression was not observed in Foxp3-Cre/Tbx21-Flp/VeDTR mice, Tbx21-Flp/VeDTR (ΔloxP) mice, and VeDTR mice.

In contrast, when examining Th1-Treg differentiation conditions, Venus expression was observed only in Foxp3-Cre/Tbx21-Flp/VeDTR mice, while Venus expression was not observed in Foxp3-Cre/VeDTR (ΔFRT) mice, Tbx21-Flp/VeDTR (ΔloxP) mice, and VeDTR mice.

From these findings, it became clear that Foxp3-Cre/Tbx21-Flp/VeDTR mice could label Th1-Treg cells with Venus only when Cre and Flp were expressed at the Foxp3 and Tbx21 gene loci.

Furthermore, to characterize the differences between Th1-Treg cells and non-Th1-Treg cells, we analyzed gene expression patterns between Venus$^+$Treg cells and Venus$^-$Treg cells in the spleen of Foxp3-Cre/Tbx21-Flp/VeDTR mice. As a result, genes showing highly suppressive function were highly expressed in resting Venus$^+$Treg cells (Example 3), and perforin and antioxidant proteins (MT3, etc.) were highly expressed in activated Venus$^+$Treg cells (Example 4).

While it is known that Th1-Treg cells infiltrate tumors, when we transplanted tumors into Foxp3-Cre/Tbx21-Flp/VeDTR mice and detected Venus-positive cells, consistent with previous findings, we were able to confirm that these cells accumulated highly in tumors compared to other organs (spleen, etc.) (Example 5).

Furthermore, when comparing tumor infiltration between Venus$^+$Treg cells and Venus$^-$Treg cells in MT3-deficient Foxp3-Cre/Tbx21-Flp/VeDTR mice transplanted with tumors, it also became clear that MT3 was necessary for the accumulation of the former in tumors (Example 6) .

Thus, in Foxp3-Cre/Tbx21-Flp/VeDTR mice, it also became clear that gene expression and functions specific to Venus$^+$ cells could be analyzed by comparing Venus$^+$ cells with Venus$^-$ cells.

Additionally, when attempting to remove Th1-Treg cells through DTR in response to diphtheria toxin administration, it became clear that Venus-positive cells in Foxp3-Cre/Tbx21-Flp/VeDTR mice decreased by 80% compared to the negative control (PBS-administered mouse group). Furthermore, tumor growth was suppressed in the diphtheria toxin-administered group compared to the negative control (Examples 7, 8). Moreover, we found that such Th1-Treg cell-specific depletion, unlike depletion of all Treg cells, does not cause autoimmunity but shows an anti-tumor effect comparable to the depletion of all Treg cells (Example 9).

Thus, in Foxp3-Cre/Tbx21-Flp/VeDTR mice, it became clear that the functions of these cells could be analyzed by specifically removing Venus+ cells.

As described above, we demonstrated that in non-human organisms such as Foxp3-Cre/Tbx21-Flp/VeDTR mice, which can express labeling genes in response to multiple characteristic gene expressions in specific cells, it becomes possible to specifically kill (remove) such cells. Furthermore, we found that by utilizing such gene expression, the gene expression and functions of the cells could be analyzed, leading to the completion of this invention.

Therefore, the present invention provides the following embodiments.

[1] A combination of DNA constructs for killing specific cells, comprising:

a first DNA construct containing a first site-specific recombinase gene operably positioned relative to a promoter of a first gene characteristic of the cells, up to an n-th DNA construct containing an n-th site-specific recombinase gene operably positioned relative to a promoter of an n-th gene characteristic of the cells; and

an (n+1)th DNA construct containing a killing gene operably positioned relative to a constitutive promoter, wherein a transcription termination sequence flanked by a pair of first recognition sequences recognized and excised by a first site-specific recombinase, up to a transcription termination sequence flanked by a pair of n-th recognition sequences recognized and excised by an n-th site-specific recombinase, are positioned between the constitutive promoter and the killing gene (where n represents a natural number of 2 or greater).

[2] The combination of DNA constructs according to [1], wherein the protein encoded by the killing gene is a protein that kills the cells in response to stimulation.

[3] The combination of DNA constructs according to [1], wherein at least one of the first to n-th site-specific recombinases is an enzyme that is activated in response to stimulation.

[4] A cell into which the combination of DNA constructs according to any one of [1] to [3] has been introduced.

[5] A non-human organism comprising the specific cell according to [4].

[6] A method for analyzing a function of specific cells, comprising the steps of:

providing stimulation to the non-human organism according to [5] to induce cell killing by the killing gene; and comparing a phenotype of the non-human organism according to [5] in which the cell according to [4] has been killed and removed by the stimulation, with a phenotype of the non-human organism according to [5] that has not been given the stimulation and in which the cell killing has not been induced.

[7] A DNA construct for killing specific cells, comprising:

a killing gene operably positioned relative to a constitutive promoter, wherein a transcription termination sequence flanked by a pair of first recognition sequences recognized and excised by a first site-specific recombinase, up to a transcription termination sequence flanked by a pair of n-th recognition sequences recognized and excised by an n-th site-specific recombinase, are positioned between the constitutive promoter and the killing gene

(where the first to n-th site-specific recombinase genes are each expressed under the control of promoters of first to n-th genes characteristic of the cells. n represents a natural number of 2 or greater).

[8] The DNA construct according to [7], wherein the protein encoded by the killing gene is a protein that kills the cells in response to stimulation.

[9] The DNA construct according to [7], wherein at least one of the first to n-th site-specific recombinases is an enzyme that is activated in response to stimulation.

[10] A specific cell into which the DNA construct according to [8] or [9] has been introduced.

[11] A non-human organism comprising the specific cell according to [10].

[12] A pharmaceutical composition containing the specific cell according to [4] or [10].

[Advantageous Effects of Invention]

According to the present invention, it becomes possible to specifically remove specific cells. Furthermore, it becomes possible to analyze the functions of the cells based on phenotypes lost through the removal of the cells.

[Brief Description of Drawings]

[Fig. 1] Fig. 1 is a figure showing an overview of genome editing for creating Foxp3-Cre/Tbx21-Flp/VeDTR (LF) mice. A shows an overview of the targeting strategy for inserting the VeDTR cassette into the Rosa26 gene locus. In the figure, "SA," "L," "stop," "F," "DTR," "IRES," "Venus," and "polyA" in the VeDTR cassette represent splice acceptor site, Cre recombinase recognition sequence (loxP sequence), transcription termination sequence (polyA addition signal sequence), Flp recombinase recognition sequence (FRT sequence), diphtheria toxin receptor (DTR) gene, internal ribosome entry site (IRES), fluorescent protein (Venus) gene, and 3' untranslated region, respectively. B shows an overview of the targeting strategy for inserting the Flp cassette into the Tbx21 gene locus. In the figure, "T-bet," "2A," "Flp," and "3UTR" in the Flp cassette represent T-bet (Tbx21) gene exon 6, sequence encoding 2A self-cleaving peptide, Flp recombinase gene, and T-bet gene 3' untranslated region, respectively.

[Fig. 2] Fig. 2 is a figure showing an overview of Cre/Flp-dependent cross-expression of Venus and DTR in Foxp3-Cre/Tbx21-Flp/VeDTR (LF) mice. The notations in the figure are the same as those in Fig. 1. As shown in A, Cre recombinase expression is controlled by the Foxp3 gene promoter. Flp recombinase expression is controlled by the Tbx21 gene promoter. Foxp3 and Tbx21 are transcription factors that define Treg cells and Th1 cells, respectively. When Cre recombinase and Flp recombinase are expressed, the removal of the two transcription termination sequences placed between these recombinase recognition sequences enables the expression of downstream DTR and Venus. Furthermore, as shown in B, when DT (diphtheria toxin) is administered to these mice, death is induced in cells that express DTR in a Cre/Flp-dependent manner.

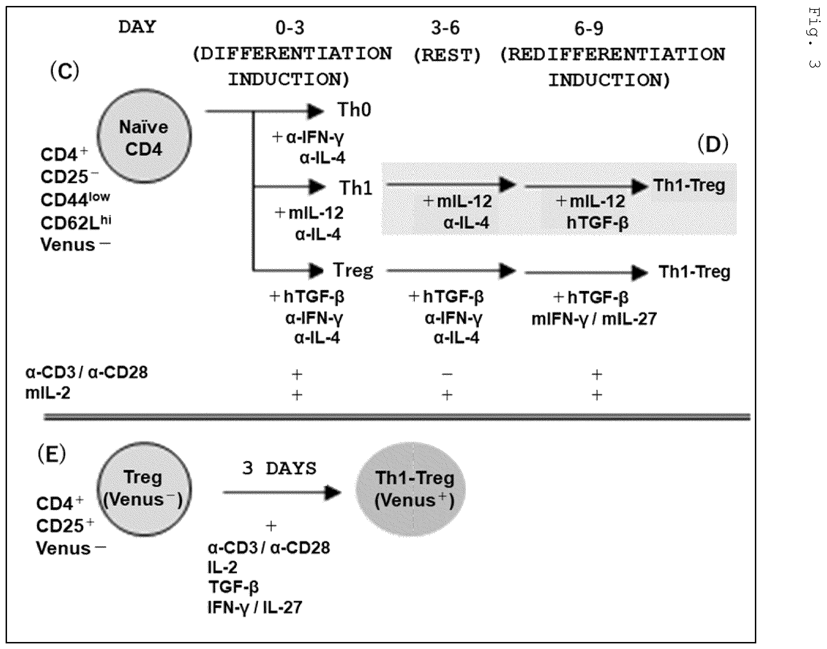

[Fig. 3] Fig. 3 is a figure showing an overview of the scheme for differentiation induction from naive CD4$^+$ T cells to Th1-Treg cells.

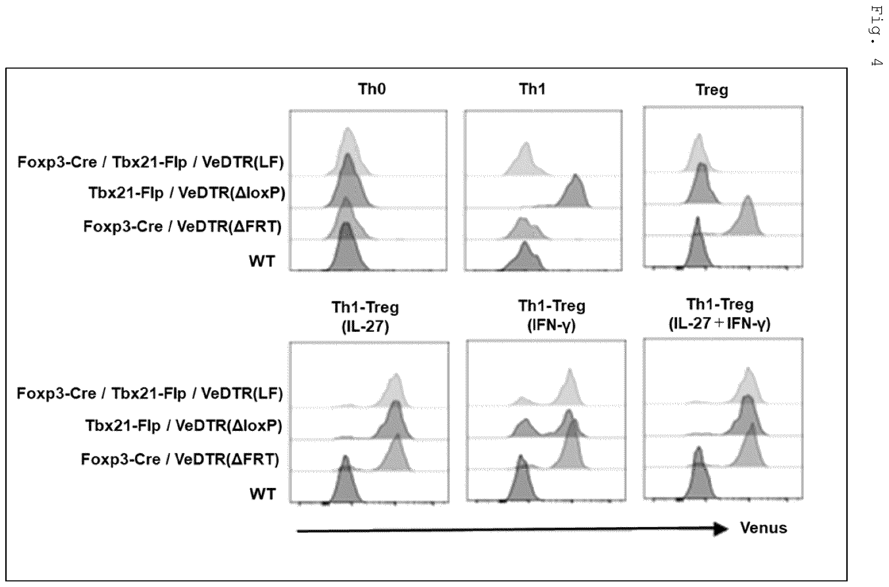

[Fig. 4] Fig. 4 is a histogram showing the analysis of Venus expression in CD4$^+$ T cells derived from mouse spleen by flow cytometry. The figure shows representative results from three independent experiments where naive CD4$^+$ T cells from wild-type (WT), Foxp3-Cre/VeDTR (ΔFRT), Tbx21-Flp/VeDTR (ΔLoxP), and Foxp3-Cre/Tbx21-Flp/VeDTR (LF) mice were cultured according to the scheme shown in (c) of Fig. 3.

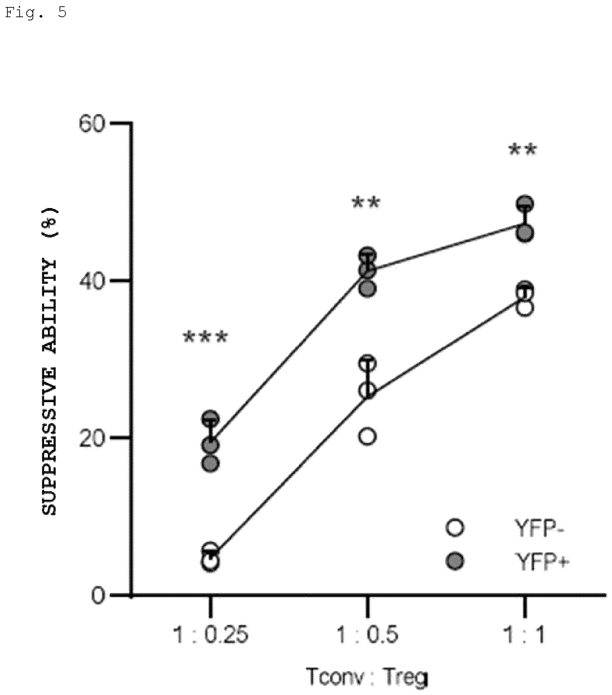

[Fig. 5] Fig. 5 is a graph showing the measurement of suppressive activity when Venus-Treg cells and Venus+Treg cells derived from the spleen of Foxp3-Cre/Tbx21-Flp/VeDTR (LF) mice were cultured with CFSE-labeled naive CD4$^+$ T cells (Tconv) for three days. Results are shown as mean with SD (n=3). Statistical significance was evaluated by unpaired two-sided Student's t-test. *** and ** indicate P < 0.001 and P < 0.01, respectively.

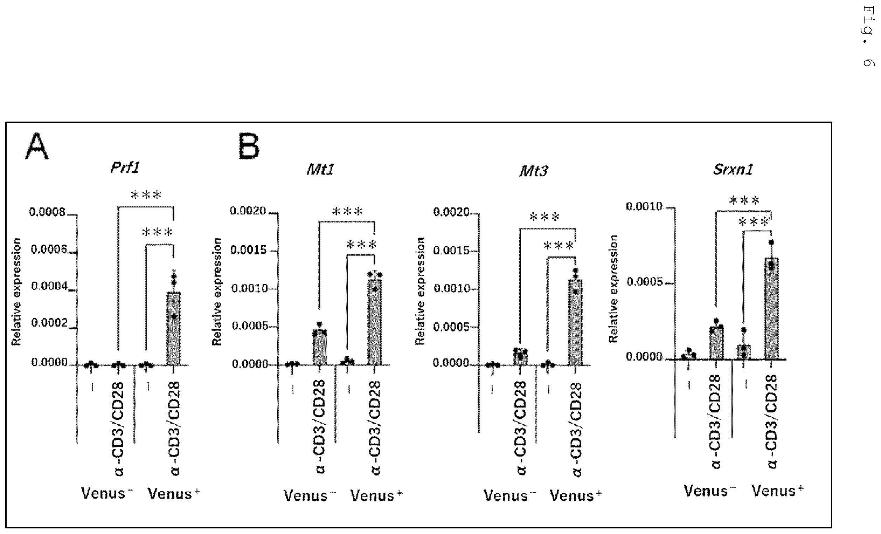

[Fig. 6] Fig. 6 is a graph showing mRNA expression analysis by quantitative PCR after Venus$^-$Treg cells and Venus$^+$Treg cells derived from the spleen of Foxp3-Cre/Tbx21-Flp/VeDTR (LF) mice were stimulated or unstimulated with α-CD3 and α-CD28 in the presence of IL-2 for three days. A shows the analysis of Prf1 mRNA expression. B shows the analysis of antioxidant protein (Mt1, Mt3, and Srxn1) mRNA expression. These results are shown as mean with SD (n=3). Statistical significance was evaluated by one-way ANOVA with post-hoc Tukey's test. *** indicates P < 0.001.

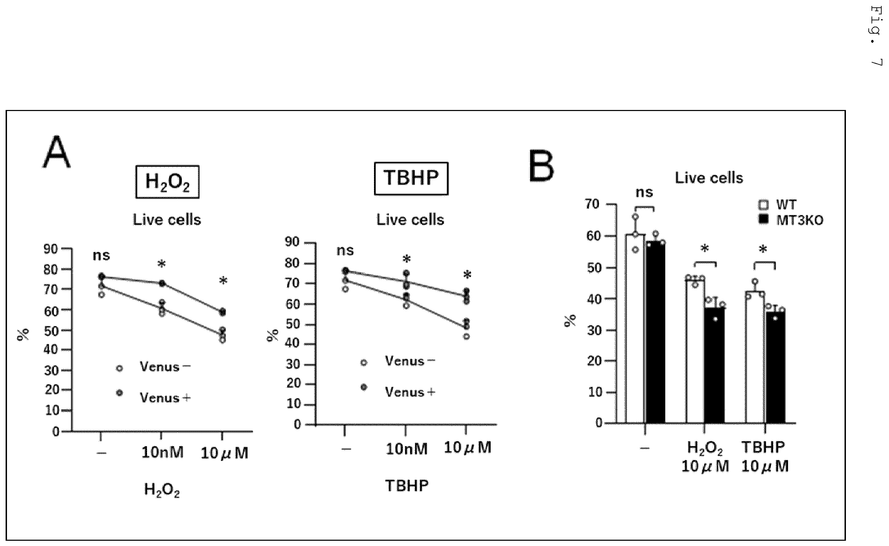

[Fig. 7] Fig. 7 is a graph showing the analysis of Treg cell resistance to oxidative stress. A shows measurement of survival rates by flow cytometry after Venus-Treg cells and Venus+Treg cells from the spleen of Foxp3-Cre/Tbx21-Flp/VeDTR (LF) mice were treated with oxidizing agents $H_2O_2$ or TBHP for 24 hours following the three-day stimulation. Results are shown as mean with SD (n=3). Statistical significance was evaluated by unpaired two-sided Student's t-test. ns indicates no significant difference, * indicates P < 0.05. B shows analysis of oxidative stress resistance in the same manner as described in A for Treg cells (CD4$^+$CD25$^+$) derived from spleens of WT and MT3-deficient mice after stimulation. Results are shown as mean with SD (n=3). Statistical significance was evaluated by unpaired two-sided Student's t-test. ns indicates no significant difference, * indicates P < 0.05.

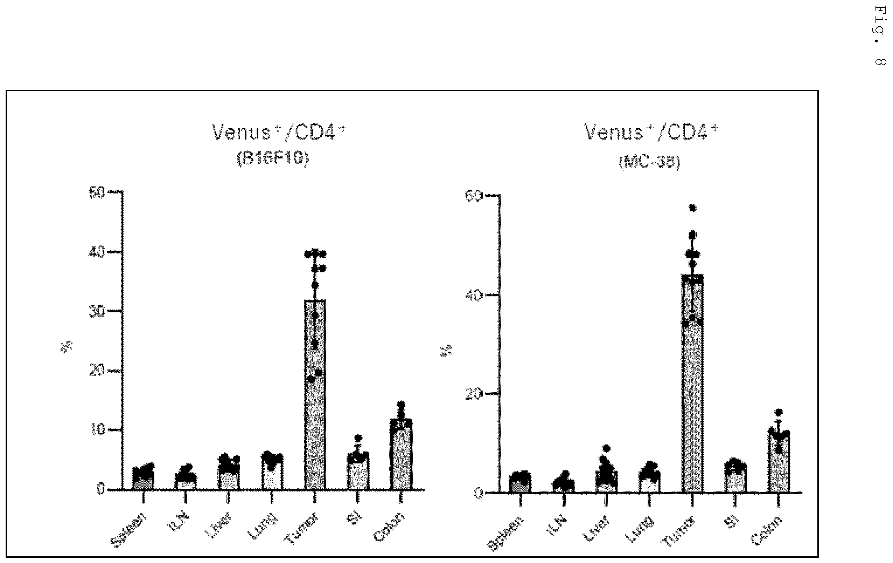

[Fig. 8] Fig. 8 is a graph showing the frequency (%) of Venus$^+$ positive cells in CD4$^+$ T cells from various tissues of B16F10-bearing mice (n=5-10, left) and MC-38-bearing mice (n=6-11, right). Results are shown as mean with SD.

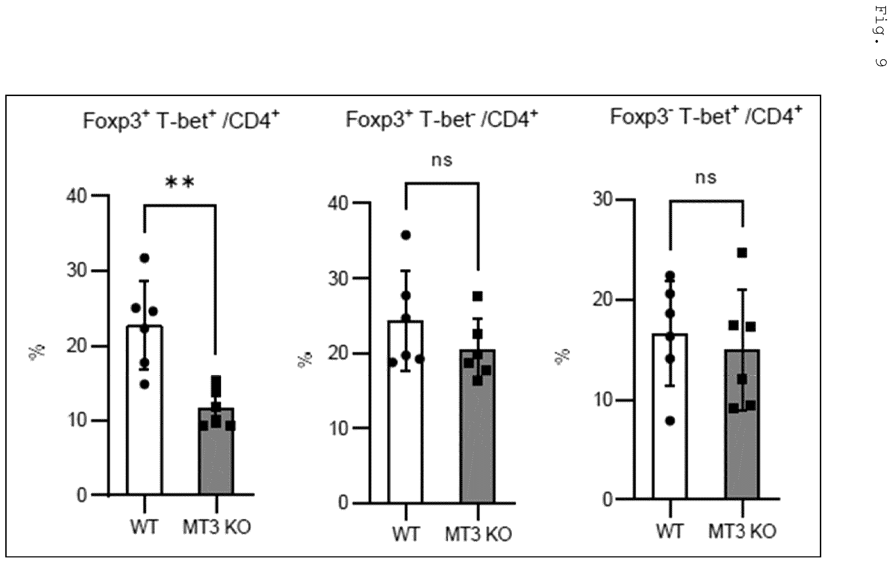

[Fig. 9] Fig. 9 is a graph showing evaluation of Foxp3 and T-bet expression in tumor-infiltrating CD4$^+$ T cells by flow cytometry in WT mice and MT3-deficient mice subcutaneously injected with B16F10. Results are shown as mean with SD (n=6). Statistical significance was evaluated by unpaired two-sided Student's t-test. ns indicates no significant difference, ** indicates P < 0.01.

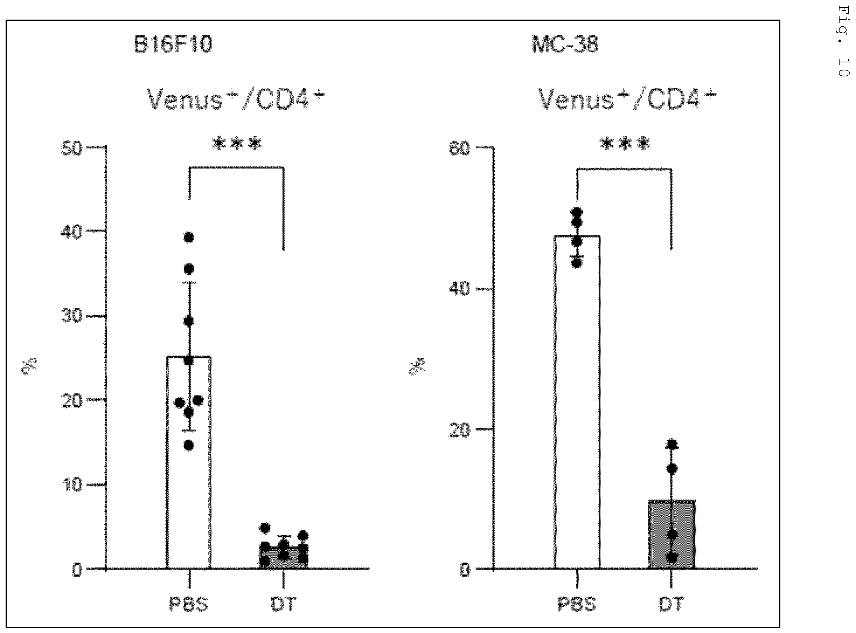

[Fig. 10] Fig. 10 is a graph showing the frequency (%) of Venus$^+$ cells among CD4$^+$ T cells in tumor-bearing mice. Foxp3-Cre/Tbx21-Flp/VeDTR (LF) mice were subcutaneously injected with B16F10 or MC-38. DT injection was started on day 10 for B16F10-bearing mice and day 14 for MC-38-bearing mice. The left graph shows the frequency of Venus$^+$ cells among tumor-infiltrating CD4$^+$ T cells in B16F10-bearing mice on days 16-18. Results are shown as mean with SD (n=8). The right graph shows the frequency of Venus$^+$ cells among tumor-infiltrating CD4$^+$ T cells in MC-38-bearing mice on day 31. Results are shown as mean with SD (n=4). In these graphs, statistical significance was evaluated by unpaired two-sided Student's t-test. ns indicates no significant difference, *** indicates P < 0.001.

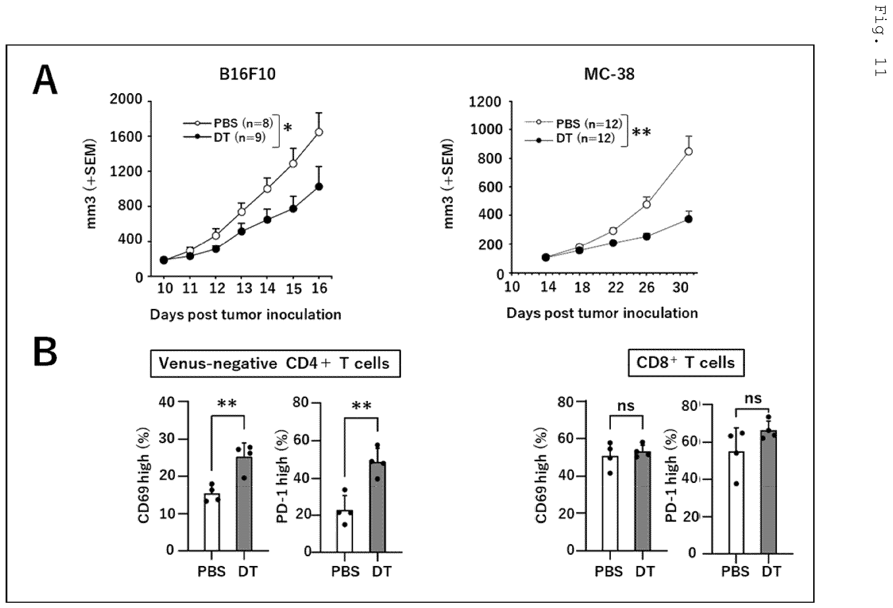

[Fig. 11] Fig. 11 is a graph showing analysis of tumor growth and CD69 and PD-1 expression in tumor-infiltrating Venus$^-$CD4$^+$ T and CD8$^+$ T cells in tumor-bearing mice described in Fig. 10. A shows analysis of B16F10 tumor growth in PBS-injected mice (n=8) and DT-injected mice (n=9). B shows analysis of tumor growth in MC-38 PBS-injected mice (n=12) and DT-injected mice (n=12). In these graphs, results are shown as mean with SD. Statistical significance was evaluated by unpaired two-sided Student's t-test. * indicates P < 0.05. ** indicates P < 0.01. C shows analysis of CD69 and PD-1 expression in tumor-infiltrating Venus⁻CD4⁺ T cells in B16F10-bearing mice. D shows analysis of CD69 and PD-1 expression in tumor-infiltrating Venus⁻CD8⁺ T cells in B16F10-bearing mice. Results are shown as mean with SD (n=4). Statistical significance was evaluated by unpaired two-sided Student's t-test. ns indicates no significant difference, ** indicates P < 0.01.

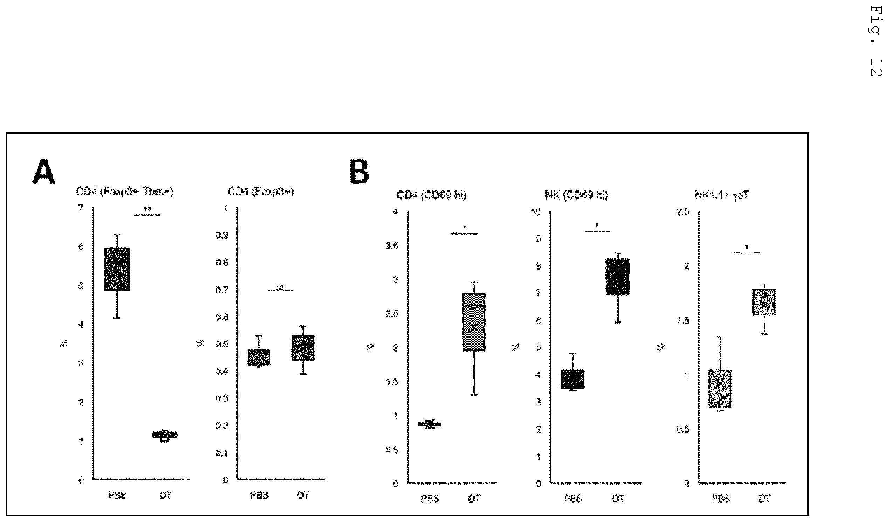

[Fig. 12] Fig. 12 is a box-and-whisker plot showing the distribution of clusters shown in mice under PBS conditions and DT conditions. Foxp3-Cre/Tbx21-Flp/VeDTR (LF) mice were subcutaneously injected with B16F1 (day 0). 100 ng DT and 200 μl PBS (DT conditions) or PBS alone (PBS conditions) were injected twice on days 10 and 11. Twenty-four hours after the second DT injection, tumor-infiltrating lymphocytes (TIL) were analyzed by CyTOF. In the figure, circles and crosses indicate median and mean values, respectively. Statistical significance was evaluated by unpaired two-sided Student's t-test. ns indicates no significant difference, * indicates P < 0.05, ** indicates P < 0.01.

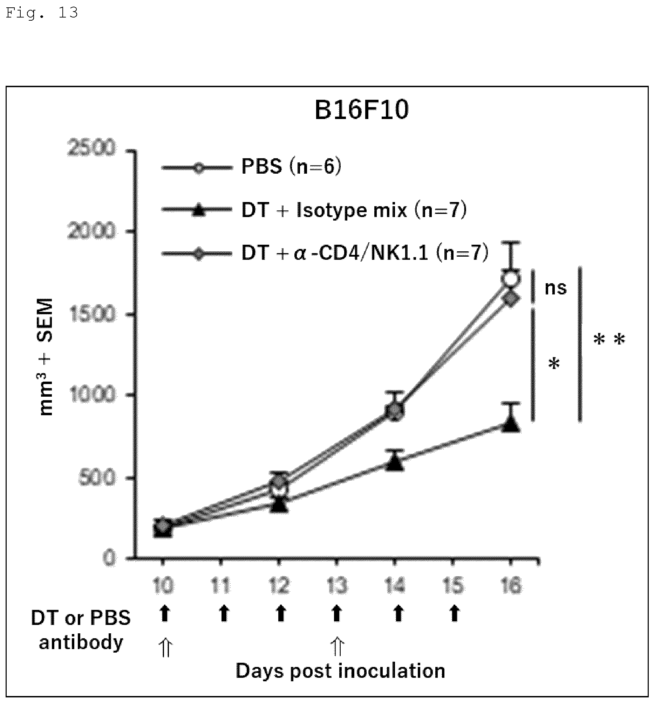

[Fig. 13] Fig. 13 is a graph showing analysis of tumor growth after intraperitoneal injection of 100 ng DT and 200 μl PBS along with antibodies into Foxp3-Cre/Tbx21-Flp/VeDTR (LF) mice subcutaneously injected with B16F10. As a control, tumor growth was analyzed in tumor-bearing mice injected intraperitoneally with PBS only instead of DT and antibodies. The horizontal axis shows the number of days after B16F10 injection and the timing of DT, PBS, and antibody injections. The vertical axis shows the volume of B16F10 tumors. Results are shown with standard error of the mean (SEM). Statistical significance was evaluated by one-way ANOVA with post-hoc Tukey's test. ns indicates no significant difference, * indicates P < 0.05, ** indicates P < 0.01.

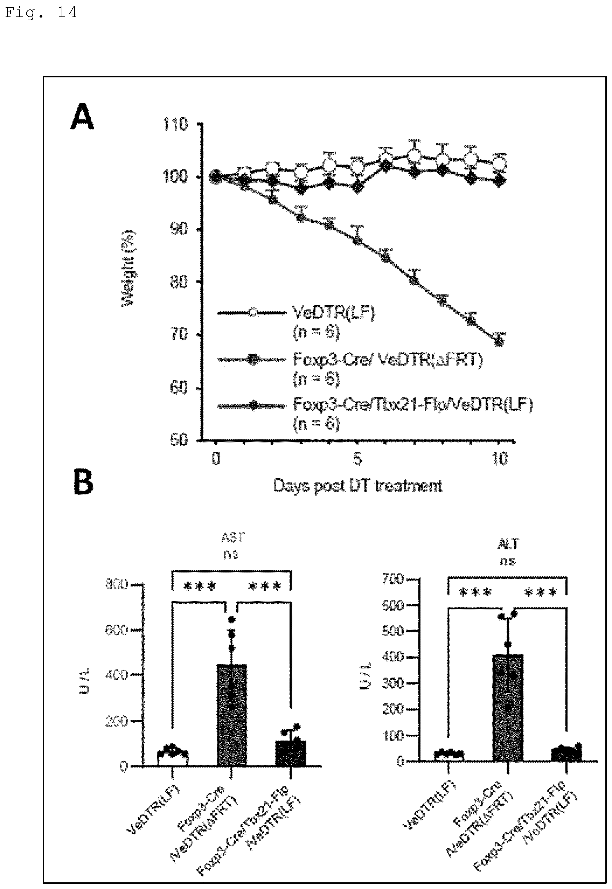

[Fig. 14] Fig. 14 is a graph showing analysis of mice in which Th1-Treg cells or all Treg cells were depleted by DT administration. VeDTR (LF) mice (n=6), Foxp3-Cre/VeDTR (ΔFRT) mice (n=6), and Foxp3-Cre/Tbx21-Flp/VeDTR (LF) mice (n=6) were intraperitoneally injected with 100 ng DT daily. A shows monitoring of body weight loss over 10 days. B shows a graph of serum AST and ALT concentrations measured 10 days after the initial DT injection. In these graphs, results are shown as mean with SD.

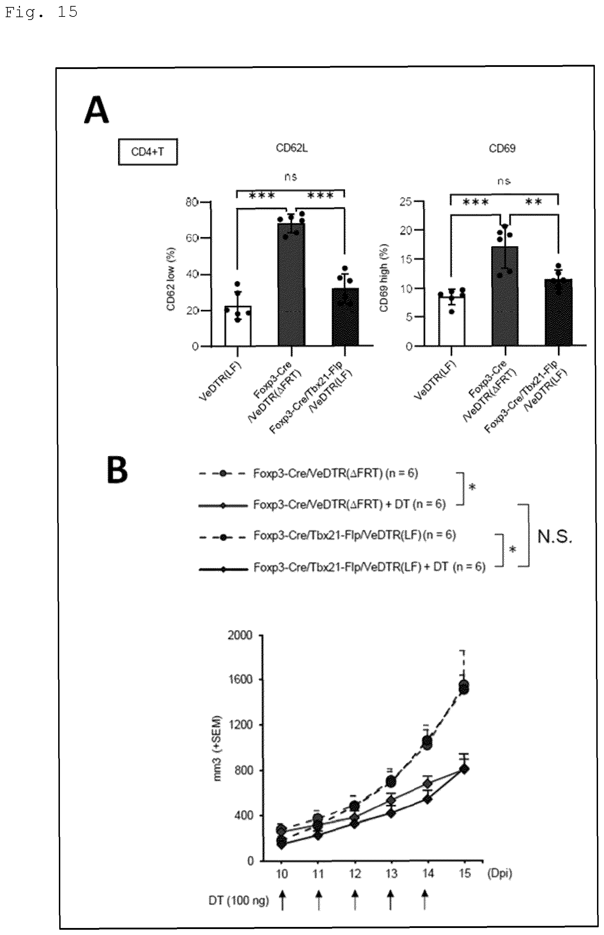

[Fig. 15] Fig. 15 is a graph showing analysis of CD62L and CD69 expression in splenic CD4⁺ T cells and tumor growth in mice described in Fig. 14. A shows measurement of CD62L and CD69 expression in splenic CD4⁺ T cells by flow cytometry. Results are shown as mean with SD (n=6). Statistical significance was evaluated by one-way ANOVA with post-hoc Tukey's test. ns indicates no significant difference, *** indicates P < 0.001, ** indicates P < 0.01. B shows analysis of B16F10 tumor growth in Foxp3-Cre/VeDTR (ΔFRT) mice and Foxp3-Cre/Tbx21-Flp/VeDTR (LF) mice. Arrows indicate the time points of DT treatment. Results are shown as mean with SD, and statistical significance at day 15 was evaluated by unpaired two-sided Student's t-test. N.S. indicates no significant difference, * indicates P < 0.05.

[Description of Embodiments]

Below, the present invention will be explained in detail according to its preferred embodiments. First, the DNA constructs of the present invention will be explained.

(DNA Constructs)

As shown in the examples described below, in the present invention, by introducing the following DNA constructs into an organism, it becomes possible to specifically kill specific cells in the organism.

A combination of

a first DNA construct containing a first site-specific recombinase gene operably positioned relative to a promoter of a first gene characteristic of specific cells, up to an n-th DNA construct containing an n-th site-specific recombinase gene operably positioned relative to a promoter of an n-th gene characteristic of the cells,

with an (n+1)th DNA construct containing a killing gene operably positioned relative to a constitutive promoter, wherein a transcription termination sequence flanked by a pair of first recognition sequences recognized and excised by a first site-specific recombinase, up to a transcription termination sequence flanked by a pair of n-th recognition sequences recognized and excised by an n-th site-specific recombinase, are positioned between the constitutive promoter and the killing gene.

In the present invention, "characteristic genes" refers to genes that are expressed in the specific cells described below but are not much expressed in other cells, and these multiple types (first to n-th) of characteristic genes enable specific identification (discrimination from other cells) of the specific cells. While "n" in the present invention can be any natural number of 2 or greater, it is usually 2 or 3. Additionally, it is desirable that the first to n-th characteristic genes are all different genes. As examples of characteristic genes, when the specific cells are Th1-Treg cells, Tbx21 (Tbet) and Foxp3 can be cited; when they are Th2-Treg cells, Gata3 and Foxp3 can be cited; when they are Th17-Treg cells, RORgt and Foxp3 can be cited; and when they are Tfh-Treg cells, Bcl6 and Foxp3 can be cited.

In the present invention, "promoter" refers to a region (sequence) responsible for gene expression control (transcription control). Furthermore, "operably positioned" relative to a promoter means that the gene exists in a position where the promoter can exert its function, namely, a position where transcription of the target gene can be performed. While such positions can be appropriately adjusted depending on the type of promoter used, etc., for example, they are typically 1000-4000 nucleotides downstream of the promoter, preferably 1500-4000 nucleotides downstream, more preferably 2000-4000 nucleotides downstream, and further preferably 3000-4000 nucleotides downstream.

"Promoters of genes characteristic of specific cells" may be promoters endogenous to the cells or promoters exogenously (artificially) introduced into the cells. When promoters of genes characteristic of specific cells are exogenously introduced into the cells, the promoters are already positioned in the present invention's DNA constructs at positions where transcription of the target site-specific recombinase genes is possible. On the other hand, when using promoters endogenous to specific cells characteristic genes as promoters, the present invention's DNA constructs do not contain the promoters, but instead adopt a form that can be inserted operably into endogenous promoters. While such insertion sites are typically preferably introns of the endogenous gene locus having the promoter, they may also be exons.

Additionally, to enable such operable insertion, typically gene targeting methods for inserting DNA into target sites of host cell genomes, or genome editing methods are used. Gene targeting methods include, for example, methods utilizing homologous recombination. Genome editing techniques are methods that modify target genes using enzymes capable of specifically cutting target sites in host cell genomes, for example, site-specific nucleases (for example, zinc finger nucleases (ZFN), transcription activator-like effector nucleases (TALEN), CRISPR-Cas9, and other DNA double-strand break enzymes). For example, fusion proteins such as ZFNs, TALENs, nuclease domain-fused PPR, etc., or methods using complexes of guide RNA (gRNA) and protein such as CRISPR-Cas9, CRISPR-Cpf1, or Target-AID can be cited. In these methods, when introducing DNA into host cells, the enzyme or its components, or nucleotides encoding the enzyme or its components are further introduced into the host cells to cut the target site, and the DNA is inserted into the target site during the cut repair process (so-called SDN-3 (Site-Directed Nuclease 3)).

Furthermore, when using such gene targeting methods or genome editing methods, in the present invention's DNA constructs, instead of including the promoter, the target gene can adopt a form where it is flanked by sequences homologous to sequences downstream of the endogenous promoter. The nucleotide number of "homologous sequences" can be any number that allows insertion through the homologous recombination or genome editing, for example, 500-7000 nucleotides (preferably 1000-5000 nucleotides, more preferably 2000-4000 nucleotides, and further preferably about 3000 nucleotides (for example, 2500-3000 nucleotides)).

In the present invention, "site-specific recombinase" can be any enzyme that recognizes specific sequences (recognition sequences) and causes recombination between the recognition sequences, and is not particularly limited, but examples include Cre, Flp, Dre, Gin, recombinase R derived from soy sauce yeast (Zygosaccharomyces rouxii), ΦC31 integrase, γδ resolvase, Tn3 resolvase, Bxb1-integrase, R4 integrase, and λ integrase.

Additionally, in the present invention, "site-specific recombinase" is not limited to enzymes that are constitutively activated such as the above enzymes but may also be enzymes that are activated in response to stimulation. Such enzymes include, for example, fusion proteins of constitutively active site-specific recombinases with hormone receptors (such as estrogen receptors). More specifically, CreERT2, FlpERT2, etc., can be cited. The ERT2 fused with Cre, Flp, etc., is an estrogen receptor modified to not bind to (endogenous) estrogen but to bind to tamoxifen. Therefore, these site-specific recombinases are activated in response to tamoxifen.

Furthermore, the site-specific recombinases of the present invention may be split partial fragments of the above enzymes. Such partial fragments can be any that reconstitute the enzyme activity when these partial fragments combine, and specific examples include N-Cre and C-Cre (Hirrlinger, J., et al., PLoS One, 2009, 4, e4286).

"Recognition sequences" can be any sequences that can be specifically recognized by the respective enzymes; for example, for Cre, loxP sequences can be cited; for Flp, FRT sequences can be cited; for Dre, rox sequences; for Gin, gix sequences; for recombinase R, Rs sequences; and for ΦC31 integrase, pseudo attP sites can be cited. The first to n-th site-specific recombinases should preferably all be different enzymes, and accordingly, the first to n-th recognition sequences should preferably all be different sequences; however, when using split partial fragments of the above enzymes, their recognition sequences will be identical (for example, when the first site-specific recombinase is N-Cre and the second site-specific recombinase is C-Cre, the first and second recognition sequences will both represent the same loxP sequence).

In the present invention, "killing gene" means a gene that encodes a protein that is expressed in specific cells and can kill the cells. Additionally, in the present invention, cell killing may be inducible, occurring in response to stimulation.

Such inducible cell killing can be achieved in response to the stimulation by making at least one of the first to n-th site-specific recombinases an enzyme that is activated in response to stimulation as described above. In such cases, examples of killing genes include diphtheria toxin A fragment (DTA).

On the other hand, when site-specific recombinases are constitutively active enzymes, as shown in the examples described below, cell killing can be made inducible by making the killing gene encode a protein that can inductively kill the cells in response to stimulation (drugs, light, temperature, etc.). Such killing genes include, for example, drug-dependent killing genes. These genes include not only genes encoding proteins that induce cell killing through drug administration but also genes encoding proteins that convert non-toxic prodrugs (drugs) into toxic substances. Examples of such drug-dependent killing genes and drug combinations include: diphtheria toxin (DT) and diphtheria toxin receptor (DTR) gene; ganciclovir or aciclovir and herpesvirus thymidine kinase (HSV-TK) gene; 6-methoxypurine arabinonucleoside and Varicella Zoster virus thymidine kinase gene; fluorouracil and E. coli cytosine deaminase gene; thioxanthine and E. coli xanthine-guanine phosphoribosyltransferase gene; non-toxic purine deoxyadenosine, adenosine, deoxyguanosine, or guanosine derivatives and E. coli or Leishmania purine nucleotide phosphorylase gene; 3-amino-1,2,4-benzotriazine 1,4-dioxide and cytochrome pla50 2B1 or cytochrome p450 reductase gene; etoposide 1-phosphate and cell-surface alkaline phosphatase gene; metronidazole or nitrofurantoin and nitroreductase gene; 1-deazapurine and N-deoxyribosyltransferase gene; metronidazole and pyruvate ferredoxin oxidoreductase gene; aminoacyl acid nitrogen mustard and carboxypeptidase G2 gene; methotrexate α-alanine and carboxypeptidase A gene; cephalosporin derivatives and lactamase gene; synthetic pentapeptide lactone precursor and actinomycin D synthetase complex gene; glucuronide derivatives (doxorubicin, etc.) and glucuronidase gene. Additionally, as combinations of drug-dependent killing genes and drugs, combinations of genes encoding cell-surface peptides and antibodies that induce cell death by recognizing the peptides can be cited. More specifically, combinations of human CD95 gene and anti-human CD95 antibody (which does not recognize mouse CD95), and human CD2 gene and anti-human CD2 antibody can be cited. In the present invention, "antibody" can be any that shows activity to induce cell death by recognizing the peptide, and its origin, type, shape, etc., are not particularly limited. Specifically, it includes non-human animal-derived antibodies (e.g., rabbit antibodies, mouse antibodies, rat antibodies, camel antibodies), human-derived antibodies, chimeric antibodies, humanized antibodies, and functional fragments of these antibodies. "Functional fragments" means parts (partial fragments) of antibodies that show the activity. Specifically, Fab, Fab', F(ab')2, variable region fragments (Fv), disulfide-linked Fv, single-chain Fv (scFv), sc (Fv) 2, diabodies, multispecific antibodies, and their polymers, etc., can be cited.

In the present invention, "constitutive promoter" is also called a structural promoter and means a promoter that can activate transcription of target genes in most cells of the organism to be introduced, at most developmental stages (times) and physiological conditions.

The constitutive promoter may be a promoter endogenous to specific cells or may be a promoter exogenously (artificially) introduced into the cells.

When the constitutive promoter is endogenous, constitutive gene promoters can be cited, and preferably, safe harbor gene promoters can be cited. Here, a "safe harbor gene" means a constitutive gene that enables stable expression of foreign genes inserted into it while ensuring that the expression of such foreign genes does not affect the expression and function of other endogenous genes, and more specific examples include ROSA26, AAVS1, CCR5, collagen, HTRP, H11, β-2 microglobulin, GAPDH, TCR, RUNX1. On the other hand, when the constitutive promoter is exogenous, examples include CAG promoter, CMV promoter, EF1α promoter, PGK promoter, UBC promoter.

Additionally, in the (n+1)th DNA construct, as in the first to n-th DNA constructs described above, when the promoter is exogenously introduced into the cells, the promoter is already positioned in the DNA construct at a position where transcription of the target site-specific recombinase gene is possible. On the other hand, when using an endogenous constitutive promoter of the cells, the present invention's DNA constructs do not contain the promoter but instead adopt a form that can be inserted operably into the endogenous promoter.

In the present invention, "transcription termination sequence" means a sequence where transcription is terminated, and examples include polyA addition signal sequence and/or terminator, but preferably multiple polyadenylation (polyA addition) signal sequences. Here, multiple means there is no particular limitation, but usually 2 or 3, and such polyA addition signal sequences also have no particular limitation; for example, polyA addition signal sequences derived from SV40 late gene or early gene, rabbit β-globin gene, bovine growth hormone gene, or human A3 adenosine receptor gene can be cited. Additionally, in the transcription termination sequence of the present invention, when multiple polyA addition signal sequences are included, they may all be derived from the same type of gene or may be derived from different genes.

In the DNA constructs of the present invention, they may include other sequences besides the various sequences described above. Such other sequences include, as shown in the examples described below, sequences that enable polycistronic expression, namely, sequences that enable simultaneous expression of two genes by one promoter. Such sequences may be sequences having self-cleaving activity, which enable simultaneous expression of two genes by activation of one promoter and, after being translated as two proteins or peptides joined via the sequence, the two proteins or peptides joined via the sequence are self-cleaved in the region of the sequence, resulting in translation of two separate proteins or peptides. Alternatively, they may be sequences that attract ribosomes and initiate a second translation from the middle of mRNA, allowing two different genes to be translated bicistronically from one mRNA. Examples of the former include "2A sequences," and examples of the latter include "IRES (internal ribosome entry site) sequences."

Additionally, when inserted into an intron of an endogenous gene locus, the DNA constructs of the present invention may further include a splice acceptor site (SA), as shown in the examples described below, to express proteins encoded by the present invention's DNA constructs.

Additionally, in the (n+1)th DNA construct of the present invention, other genes may also be operably positioned relative to the constitutive promoter in addition to the killing gene described above. "Other genes" are not particularly limited, but examples include labeling genes such as Venus shown in the examples described below.

In the present invention, "labeling gene" can be any gene that encodes a labeling protein that is expressed in specific cells and can be detected; examples include fluorescent protein genes, luminescent enzyme genes, chromogenic enzyme genes. Examples of fluorescent protein genes include Venus gene, YFP (yellow fluorescent protein) gene, GFP (green fluorescent protein) gene, RFP (red fluorescent protein) gene, aequorin gene. Examples of luminescent enzyme genes include luciferase gene. Examples of chromogenic enzyme genes include β-glucuronidase (GUS) gene, β-galactosidase gene, alkaline phosphatase gene, SEAP gene.

The form of the DNA constructs of the present invention can be either linear or circular, and examples include viral vectors, plasmid vectors, episomal vectors, artificial chromosome vectors, transposon vectors.

Examples of viral vectors include retroviral vectors such as lentivirus, Sendai virus vector, adenovirus vector, adeno-associated virus vector, herpesvirus vector, vaccinia virus vector, poxvirus vector, poliovirus vector, Sindbis virus vector, rhabdovirus vector, paramyxovirus vector, orthomyxovirus vector.

Examples of plasmid vectors include animal cell expression plasmid vectors such as pcDNA3.1, pA1-11, pXT1, pRc/CMV, pRc/RSV, pcDNAI/Neo.

Episomal vectors are vectors capable of autonomous replication outside chromosomes. Specific means of using episomal vectors are known (see Yu et al., Science, 2009, 324, pp. 797-801). Examples of episomal vectors include vectors containing sequences necessary for autonomous replication derived from EBV, SV40, etc., as vector elements. Vector elements necessary for autonomous replication specifically include a replication origin and a gene encoding a protein that binds to the replication origin to control replication; for example, in EBV, replication origin oriP and EBNA-1 gene, in SV40, replication origin ori and SV40 LT gene can be cited.

Examples of artificial chromosome vectors include YAC (Yeast artificial chromosome) vector, BAC (Bacterial artificial chromosome) vector, PAC (P1-derived artificial chromosome) vector.

(Specific Cells, Non-Human Organism)

As shown in the examples described below, the present invention can also provide cells into which the above combination of DNA constructs has been introduced (also referred to as "specific cells of the present invention" below), and non-human organisms containing the cells (also referred to as "non-human organisms of the present invention" below).

In the present invention, "specific cells" refers to any target cells to be removed and is not particularly limited, but as shown in the examples described below, subsets of immune cells (Th1 cells, Th2 cells, Th17 cells, Treg cells, Tfh cells, B cells, macrophages, dendritic cells, NK cells) can be cited as suitable examples.

In the present invention, there is no particular limitation on non-human organisms; examples include mammals such as rodents including mice and rats, cattle, horses, pigs, sheep, monkeys, dogs, and cats, birds such as chickens, and plants such as wheat, rice, cypress, cedar, and roses.

There is no particular limitation on the method for producing non-human organisms containing the specific cells, but as shown in the examples described below, non-human organisms containing the specific cells can be produced by introducing the present invention's DNA constructs, along with genome editing systems as needed, into early embryos, transplanting the embryos into the uterus of non-human organisms, allowing development, and crossing the obtained individuals.

Methods for preparing the specific cells of the present invention include isolation from the non-human organisms. There is no particular limitation on such isolation methods, and those skilled in the art can adopt and perform suitable methods according to the cell type. For example, methods for collection by flow cytometry using the expression of genes characteristic of specific cells as markers can be cited. Additionally, when a labeling gene is positioned in the (n+1)th DNA construct as described above, as shown in the examples described below, efficient collection becomes possible using the gene as a marker for flow cytometry.

Furthermore, the "cells" specific to the present invention can be prepared without going through non-human organisms as described above. For example, they can be prepared by directly introducing the combination of DNA constructs of the present invention into specific cells. They can also be prepared by introducing the combination of DNA constructs of the present invention into precursor cells or stem cells of specific cells and inducing their differentiation. There is no particular limitation on methods for introducing DNA into such cells; for example, known methods such as lipofection, microinjection, calcium phosphate method, DEAE-dextran method, electroporation method, and particle gun method can be used. Additionally, such differentiation induction can also be performed by those skilled in the art adopting appropriate known techniques based on the cell type.

(Method for Analyzing Functions of Specific Cells)

As shown in the examples described below, by using the above combination of DNA constructs, it becomes possible to kill (remove) specific cells, enabling analysis of specific cells as follows. Therefore, the present invention also provides the following methods.

(1) A method for analyzing functions of specific cells comprising the steps of: providing the stimulation to the non-human organism of the present invention to induce cell killing by the killing gene; and comparing the phenotype of the non-human organism of the present invention in which the specific cells of the present invention have been killed and removed by the stimulation with the phenotype of the non-human organism of the present invention that has not been given the stimulation.
(2) A method for analyzing functions of specific cells comprising a step of: detecting and comparing the phenotype of the specific cells of the present invention with the phenotype of other cells in the non-human organism of the present invention.
(3) A method for screening genes characteristic of specific cells comprising a step of: detecting and comparing gene expression in the specific cells of the present invention with gene expression in other cells in the non-human organism of the present invention.

The "non-human organism of the present invention" used in the above analysis methods is as described above, but may further be a non-human organism that has been administered specific drugs or in which specific genes have been knocked out or forcibly expressed. Such non-human organisms can be used as model animals for diseases, etc., and by analyzing the functions of specific cells in such model animals, it becomes possible to analyze correlations between the cells and the diseases.

Methods for "providing stimulation" to the non-human organism of the present invention are not particularly limited, and those skilled in the art can select and perform appropriate methods from known techniques according to the type of stimulation to be given. For example, when the stimulation is a drug, it can be performed by oral or non-oral administration of the drug to the non-human organism. Non-oral administration includes, for example, intravenous administration, intra-arterial administration, intraperitoneal administration, subcutaneous administration, intradermal administration, airway administration, rectal administration, intramuscular administration, infusion administration, direct administration (for example, local administration to specific cells or tissues where they exist).

"Other cells" that serve as comparison controls in the methods of the present invention can be any cells different from specific cells with no particular limitation, but examples include cell subsets different from specific cells (more specific examples include subsets of Treg cells other than Th1-Treg cells when specific cells are Th1-Treg cells).

Additionally, in the methods of the present invention, there are no particular limitations on the "phenotype" to be targeted and its detection methods, and those skilled in the art can select phenotypes to be targeted according to the specific cells and other cells to be compared, and select detection methods to match them to perform analysis.

In the method for screening genes characteristic of specific cells of the present invention, the gene expression to be detected may be at the transcription level (RNA) or at the translation level (protein). Additionally, the genes to be detected may be one or several specific genes, or as shown in the examples described below, may be comprehensive gene groups.

While there is no particular limitation on such detection methods, when analyzing at the transcription level, examples include PCR (RT-PCR, real-time PCR, quantitative PCR), DNA microarray analysis, Northern blotting, next-generation sequencing methods (sequencing-by-synthesis (e.g., sequencing using Solexa genome analyzer or HiSeq (registered trademark) 2000 manufactured by Illumina), pyrosequencing (e.g., sequencing using Sequencer GS LX or FLX manufactured by Roche Diagnostics (454) (so-called 454 sequencing)), ligase reaction sequencing (e.g., sequencing using SOLiD (registered trademark) or 5500xl manufactured by Life Technologies)), T-RFLP method, bead array method, in situ hybridization, dot blot, RNase protection assay method, mass spectrometry.

Additionally, when analyzing at the translation level, examples include methods using antibodies for detection (immunological methods) such as ELISA, immunoblotting, antibody array analysis, immunohistochemical staining, flow cytometry, imaging cytometry, radioimmunoassay, immunoprecipitation method, and mass spectrometry.

(Pharmaceutical Composition)

By introducing the combination of DNA constructs of the present invention, it becomes possible to remove specific cells in vivo in response to the above stimulation. Therefore, the present invention can also provide an embodiment of a composition for treating or preventing diseases caused by specific cells, with the DNA constructs of the present invention as active ingredients. In such cases, treatment etc. becomes possible by directly administering or delivering the combination of DNA constructs to the affected area where specific cells exist.

Additionally, the DNA constructs and specific cells of the present invention can be used not only for such in vivo treatment but also for ex vivo treatment. For example, when performing gene therapy on specific cells derived from patients, by introducing the combination of DNA constructs of the present invention, it becomes possible to remove or reduce the cells in response to the above stimulation after returning them to the patient's body. As a more specific example, in CAR-T cell therapy, to suppress side effects etc., it becomes possible to suppress their immunological effect by removing or reducing the T cells in response to the above stimulation. Therefore, the present invention can also provide a pharmaceutical composition having as an active ingredient at least one of the first to (n+1)th DNA constructs of the present invention (for example, the (n+1)th DNA construct of the present invention, the combination of the first to (n+1)th DNA constructs of the present invention), or cells into which they have been introduced.

The composition of the present invention can be formulated according to known pharmaceutical methods depending on the type of active ingredient (DNA constructs, cells). In such formulation, it can be appropriately combined with pharmacologically acceptable carriers, specifically, physiological saline, sterile water, media, plant oils, antibiotics, solvents, excipients, bases, emulsifiers, suspending agents, surfactants, stabilizers, vehicles, preservatives, binders, diluents, isotonizing agents, anesthetic agents, bulking agents, disintegrants, buffers, coating agents, lubricants, thickeners, solubilization aids, or other additives.

There is no particular limitation on the diseases targeted by the pharmaceutical composition of the present invention; examples include tumors (solid tumors and hematological tumors such as leukemia), autoimmune diseases. Additionally, while the administration route of the pharmaceutical composition of the present invention is not particularly limited, examples include intravenous injection, intra-arterial injection, portal vein injection, intradermal injection, subcutaneous injection, intramuscular injection, and intraperitoneal injection. It may also be local administration rather than systemic administration. The dosage schedule, including dosage amount, can be appropriately adjusted by those skilled in the art considering the type of active ingredient or pharmaceutical composition, target (patient) gender, age, weight, disease state, etc. It may be single administration or multiple administrations continuously or periodically.

As explained above, while the present invention has been described through its preferred embodiments, the present invention is not limited to the above embodiments. For example, as described above, the non-human organism of the present invention can be produced by crossing the above individuals. Therefore, the present invention also provides the following individuals (non-human organisms) or specific cells that retain each DNA construct, in addition to non-human organisms retaining the combination of DNA constructs of the present invention.

Non-human organisms containing specific cells into which at least one of the first to n-th DNA constructs has been introduced.

Non-human organisms containing specific cells into which the (n+1)th DNA construct has been introduced. Specific cells into which at least one of the first to n-th DNA constructs has been introduced.

Specific cells into which the (n+1)th DNA construct has been introduced.

[Examples]

Below, the present invention will be explained more specifically through examples, but the present invention is not limited to these examples. Additionally, these examples were conducted using the following materials and methods.

(Mice, Cells)

Regarding CD45.1 C57BL/6N congenic mice and Foxp3-Cre mice, refer to Wing, K. et al. Science (2008) 322, 271-275 and Sasaki, I. et al. Blood (2012) 120, 4733-4743. All animal experiments were approved by the Animal Research Committee of the Research Institute for Microbial Diseases, Osaka University.

B16F10 and MC-38 were maintained in DMEM (manufactured by Nacalai Tesque) and RPMI1640 (manufactured by Nacalai Tesque), respectively. Both culture media were used with the addition of 10% heat-inactivated FCS, 100 U/ml penicillin/streptomycin, and 50 mM 2-ME.

(Creation of VeDTR Mice, Tbx21-Flp Mice, and MT3-deficient Mice)

Guide RNAs (gRNAs) targeting Rosa26, Tbx21, and Mt3 were prepared as T7 transcripts amplified using primer sets for each gene and KOD FX NEO (manufactured by TOYOBO). Cas9 mRNA was generated by in vitro transcription (IVT) using the mMESSAGE mMACHINE T7 ULTRA kit (manufactured by Life Technologies) and template amplified by PCR using pEF6-hCas9-Puro and primers for Cas9, followed by gel purification. Furthermore, the synthesized gRNA and Cas9 mRNA were purified using the MEGAclear kit (Life Technologies).

To obtain VeDTR mice, Tbx21-Flp mice or Mt3-deficient mice and CD45.2 C57BL/6N female mice (6 weeks old) were superovulated and mated with CD45.2 C57BL/6N stud males. Fertilized one-cell stage embryos were collected from oviducts and injected with 100 ng/μl Cas9 mRNA, 50 ng/μl gRNA, and 50 ng/ml targeting vector for VeDTR or Tbx21-Flp mice into the pronucleus or cytoplasm. The genome editing system-injected viable embryos were transferred to the oviducts of pseudopregnant ICR females at 0.5 days post-coitus. Male offspring carrying mutations were mated with CD45.2 C57BL/6N female mice and tested for germline transmission.

To create VeDTR mice, a 3.6 kb fragment containing a 500 bp fragment of the intron between exon 1 and exon 2 of the Rosa26 gene was amplified by PCR and cloned into the pBlueScript vector. A bicistronic reporter cassette containing the EMCV IRES followed by the human DTR sequence was cloned 5' to the Venus cDNA (Clontech). Two tandem polyA addition signal sequences (also called LSL or FSF) flanked by two loxP or FRT sequences following the splice acceptor site (SA) were artificially synthesized (synthesized at FASMAC). The SA-LSL-FSF sequence was ligated with the DTR-IRES-Venus cassette. Furthermore, the SA-LSL-FSF-DTR-IRES-Venus cassette was linked to Rosa26_LA and Rosa26_RA fragments. The resulting targeting vector was gel-purified and used for injection into embryos containing Rosa26_gRNA and Cas9 mRNA.

To create Tbx21-Flp mice, a 1.4 kb fragment containing exon 6 and the 3' untranslated sequence of the Tbx21 gene was amplified by PCR and cloned into the pBlueScript vector. The P2A peptide sequence containing mammalian cell codon-optimized Flp recombinase cDNA was artificially synthesized (synthesized at FASMAC) and inserted just before the stop codon in exon 6 of Tbx21. The targeting vector was gel-purified and used for injection into embryos containing Tbx21_gRNA and Cas9 mRNA.

(Cell Preparation from Mice)

Splenocytes were prepared by mashing tissues in PBS on a 70 $\mu$m cell strainer using a 5 ml syringe plunger. Furthermore, they were recovered by centrifugation at 2000 rpm for 5 minutes, then suspended in ACK buffer and held at room temperature for 2 minutes to lyse red blood cells. After ACK treatment, cells were washed and filtered through a cell strainer.

To prepare single-cell suspensions from tumor tissues, tumors were harvested from tumor-bearing mice, minced to 2-3 mm by laser in HBSS, then Collagenase D (1 mg/ml), Dispase II (80 $\mu$g/ml), and DNase I (20 $\mu$g/ml) were added to HBSS containing tumor pieces, and incubated at 37°C for 60 minutes with shaking (160 rpm). After digestion, cells passed through a 70 $\mu$m cell strainer were treated with ACK as described for splenocytes, then suspended in 40% Percoll (manufactured by Sigma-Aldrich) in HBSS and centrifuged at 2,380 $\times$ g for 20 minutes to remove floating debris. The precipitates were washed with HBSS. The prepared cells were suspended in appropriate buffer for each experiment. Collagenase D, Dispase II, and DNase I were purchased from Roche.

(Flow Cytometry and Cell Selection)

For surface staining, cells were stained with antibodies in PBS containing 2% BSA on ice for 15 minutes, then washed twice with PBS containing 2% BSA. Intracellular staining was performed using the Foxp3/Transcription Factor Staining Buffer Set (manufactured by eBioscience) according to the manufacturer's instructions. FACS Aria III (manufactured by BD) was used for data acquisition and cell sorting. Precision Count Beads (manufactured by BioLegend) were used to obtain cell numbers in some experiments. Acquired data was analyzed using FlowJo ver. 10.8.0 (manufactured by BD).

(In Vitro Differentiation of Naive CD4$^+$ T Cells into Th1-Treg Cells)

The differentiation scheme for naive CD4$^+$ T cells is shown in Fig. 3. Briefly, splenic naive CD4$^+$ T cells (Lin (B220, CD8a, CD11b, CD11c, NK1.1)$^-$ CD4$^+$ Venus$^-$ CD25$^-$CD62L$^{high}$ CD44$^{low}$) recovered from Foxp3-Cre/Tbx21-Flp/VeDTR (LF) mice were stimulated with pre-bound $\alpha$-CD3 and $\alpha$-CD28 in RPMI1640 culture medium containing mIL-2. Several cytokines and neutralizing antibodies shown in Fig. 3 were additionally added to the cultures. Cells were cultured for 3 days, at which point they were either analyzed or rested for an additional 3 days in fresh medium containing cytokines and neutralizing antibodies in the absence of $\alpha$-CD3/$\alpha$-CD28 stimulation. After 6 days of culture, cells were washed and restimulated with pre-bound $\alpha$-CD3 and $\alpha$-CD28 in the presence of cytokines for an additional 3 days.

The concentrations of antibodies and cytokines were as follows:
a-CD3 (5 $\mu$g/ml), a-CD28 (2 $\mu$g/ml), a-IL-4 (10 $\mu$g/ml), a-IFN-$\gamma$ (10 $\mu$g/ml), mIL-2 (20 ng/ml), mIL-12 (10 ng/ml), mIL-27 (50 ng/ml), mIFN-$\gamma$ (50 ng/ml), hTGF-$\beta$ (5 ng/ml). $\alpha$-CD3 and $\alpha$-CD28, and mIL-27 were purchased from BioLegend. mIFN-$\gamma$, mIL-2, mIL-12, and hTGF-$\beta$ were purchased from PeproTech. $\alpha$-IFN-$\gamma$ antibody and $\alpha$-IL-4 antibody were purchased from Bio X Cell.

(In Vitro Conversion of Naturally Occurring Treg Cells to Th1-Treg Cells)

Venus-Treg cells (Lin$^-$CD4$^+$CD25$^+$Venus$^-$) from Foxp3-Cre/Tbx21-Flp/VeDTR (LF) mice were recovered and stimulated for 3 days with pre-bound $\alpha$-CD3 and $\alpha$-CD28 in RPMI1640 containing mIL-2, hTGF-$\beta$ in the presence of mIFN-$\gamma$ and/or mIL-27.

(RNA-seq Analysis)

For RNA-seq analysis, total RNA was extracted using TRI Reagent (manufactured by Sigma-Aldrich) according to the manufacturer's instructions. Full-length cDNA was prepared using the SMART-Seq HT kit (Takara Bio) according to the manufacturer's instructions. Illumina libraries were prepared using the NextEra DNA Library Preparation Kit (manufactured by Illumina) following the instructions in the SMARTer kit manual. Sequencing was performed on an Illumina NovaSeq 6000 sequencer (manufactured by Illumina) in 101-base single-end mode. Sequenced reads were mapped to the mouse reference genome sequence (mm10) using TopHat v2.0.13 in combination with Bowtie2 ver. 2.2.3 and SAMtools ver. 0.1.19. Fragments per kilobase of exon per million mapped fragments (FPKM) were calculated using Cufflinks version 2.2.1. Raw count data were analyzed using iDEP.92 {see Ge, S.X. et al. BMC Bioinformatics (2018) 19, 534.} and GSEA software ver. 4.1.0 {Subramanian, 2005 #81}. Genes with FDR < 0.05 and |log2FC| > 1 were considered differentially expressed genes (DEGs). For GSEA, gene sets were obtained from published data. Heatmaps visualizing gene expression levels normalized on a raw min to raw max scale for each gene were generated using Morpheus (software.broadinstitute.org/morpheus). Gene ontology (GO) terms were obtained from MGI (www.informatics.jax.org/).

(Quantitative PCR)

For quantitative PCR, total RNA was extracted using the RNeasy Mini Kit (manufactured by Qiagen), then RNA was reverse transcribed using the Verso cDNA Synthesis Kit (manufactured by Thermo Scientific) according to the manufacturer's instructions. Quantitative PCR was performed using GoTaq qPCR Master Mix (manufactured by Promega) and CFX Connect (manufactured by Bio-Rad). mRNA expression was normalized to b-actin mRNA expression.

(In Vitro Treg Suppression Assay)

Splenic naive CD4$^+$ T cells (Tconv cells) were recovered from CD45.1 congenic mice and labeled using the CellTrace CFSE Cell Proliferation Kit (manufactured by Invitrogen) according to the manufacturer's instructions. CFSE-labeled Tconv cells were cultured with Venus$^-$Treg cells or Venus$^+$Treg cells isolated from Foxp3-Cre/Tbx21-Flp/VeDTR (LF) mice in the presence of Dynabeads Mouse T-Activator CD3/CD28 (manufactured by Gibco) and mIL-2. After 3 days of culture, CFSE intensity in Tconv cells was measured using flow cytometry, and suppressive activity (%) was calculated using the following formula:

$$100 \times (1 - (\text{frequency of CFSE}^{low} \text{ in Tconv treated with Treg}) / (\text{frequency of CFSE}^{low} \text{ in Tconv alone})).$$

Additionally, CD45.1 congenic mice were used to distinguish between Tconv (CD45.1) and Treg cells (CD45.2).

(Oxidative Stress Assay)

Venus$^-$Treg cells (Lin$^-$CD4$^+$CD25$^+$Venus$^-$) and Venus$^+$Treg cells (Lin$^-$CD4$^+$CD25$^+$Venus$^+$) were isolated from Foxp3-Cre/Tbx21-Flp/VeDTR (LF) mice, and Treg cells (Lin$^-$CD4$^+$CD25$^+$) were isolated from wild-type (WT) mice and MT3 KO mice. The isolated cells were stimulated with pre-bound $\alpha$-CD3 and $\alpha$-CD28 in RPMI1640 containing mIL-2. After 3 days of culture, cells were washed and cultured for an additional 24 hours with mIL-2 in the presence or absence of $H_2O_2$ (10 nM - 10 $\mu$M) or TBHP (10 nM - 10 $\mu$M). Then, cells were stained with DAPI, and viability was determined using flow cytometry.

(Tumor Transplantation and Diphtheria Toxin (DT) Treatment)

Age- and sex-matched mice were shaved on their backs, and on day 0, 100 $\mu$l of PBS containing $3 \times 10^5$ B16F10 cells or $5 \times 10^5$ MC-38 cells was subcutaneously injected into the shaved area (day 0). For DT treatment, tumor-bearing mice were intraperitoneally injected with 200 $\mu$l PBS containing 100 ng DT (manufactured by Millipore). DT treatment was initiated on day 10 for B16F10-bearing mice and day 14 for MC-38-bearing mice. DT treatment was performed daily from the first day of DT treatment.
Each antibody was intraperitoneally injected at 200 $\mu$g per dose. Antibodies used were $\alpha$-CD4 antibody (GK1.5) and $\alpha$-NK1.1 antibody (PK136), and their isotype controls (LTF-2 and C1.18.4), purchased from Bio X Cell. Tumor volume was measured using digital calipers and calculated using the following formula:

$$(\text{short diameter})^2 \times \text{long diameter} \times 0.52.$$

When the calculated tumor volume exceeded 2500 mm$^3$, mice were euthanized within 24 hours.

(Histological and Immunohistochemical Staining)

For histological staining, livers were fixed by transcardial perfusion with 10% formalin. Additionally, lungs were collected and fixed by degassing in 10% formalin. Subsequently, both liver and lung were post-fixed in 10% formalin at 4°C for 48 hours. The fixed tissues were embedded in paraffin and sectioned at 5 μm using REM-710 (manufactured by Yamato). Sections were stained with hematoxylin and eosin and mounted using Mount-Quick (manufactured by Daido Sangyo). Image acquisition was performed using FSX100 (manufactured by Olympus).

For immunohistochemical staining, tumors were fixed by transcardial perfusion with 4% paraformaldehyde (PFA) and post-fixed with 4% PFA at 4°C for 2 hours. The fixed tumors were immersed in 30% sucrose at 4°C overnight, embedded in FSC 22 Frozen Section Media (manufactured by Leica), and sectioned at 15 μm using CM1860 UV (manufactured by Leica). Sections were incubated in blocking buffer (PBS containing 0.1% BSA, 1% mouse serum, 1% donkey serum) at room temperature for 1 hour. Then, sections were primary stained at 4°C in blocking buffer with Alexa594-conjugated a-CD4 antibody (GK1.5) (manufactured by BioLegend) and Goat-α-DTR polyclonal antibody (manufactured by R&D Systems) overnight. Sections were washed three times in PBS, then secondary stained with CF488A-conjugated Donkey α-Goat IgG H&L (manufactured by BIOTIUM) in blocking buffer at room temperature for 1 hour. Stained sections were washed three times in PBS and mounted with DAPI (manufactured by Invitrogen) in ProLong Diamond Antifade Mountant. Image acquisition was performed using FV3000 (manufactured by Olympus).

(Preparation of Tumor-Infiltrating Immune Cells for CyTOF (registered trademark))

For CyTOF analysis of tumor-infiltrating immune cells, tumors were digested using the Tumor Dissociation Kit, mouse (manufactured by Miltenyi) and gentleMACS Octo Dissociator with Heater (manufactured by Miltenyi). Preset programs 37C_m_TDK_1 for B16F10 and 37C_m_TDK_2 for MC-38 were applied. After digestion, cells were treated with ACK buffer, then dead cells were removed using the Dead Cell Removal Kit (manufactured by Miltenyi). Finally, CD45$^+$ cells were observed using CD45 (TIL) MicroBeads, mouse (manufactured by Miltenyi) and LS Column (manufactured by Miltenyi). All procedures were performed according to the manufacturer's instructions.

(Preparation of Metal-Conjugated Antibodies for CyTOF)

Maxpar MCP9 Antibody Labeling Kit and X8 Antibody Labeling Kit were used according to the manufacturer's instructions for cadmium labeling and lanthanoid metal labeling of antibodies, respectively. For platinum labeling of α-CD45 antibodies, Cell-ID Cisplatin-194Pt, Cisplatin-195Pt, Cisplatin-196Pt, and Cisplatin-198Pt were used following Mei, H.E. et al. Cytometry A (2016) 89, 292-300. The above kits, reagents, and all metal pre-conjugated antibodies were purchased from Fluidigm.

(CyTOF Cell Staining)

First, each sample was barcoded using FC block antibodies and the cell size marker wheat germ agglutinin (WGA)-FITC with different metal-conjugated α-CD45 antibodies in CyFACS buffer (PBS containing 0.1% BSA, 2 mM EDTA, and 0.01% sodium azide) {see Stern, A.D. et al. Cytometry A (2017) 91, 14-24.}. After barcoding, samples were washed twice with CyFACS buffer and pooled. Pooled samples were stained with metal-conjugated antibody cocktail for surface proteins in CyFACS buffer at room temperature for 45 minutes. Then, samples were washed twice with CyFACS buffer and stained with PBS containing zirconium(IV) chloride at room temperature for 5 minutes to distinguish live cells from dead cells {see Devine, R.D. et al. Cytometry A (2021) 99, 1042-1053.}. Then, fixation and permeabilization were performed using the Foxp3/Transcription Factor Staining Buffer Set according to the manufacturer's instructions. Subsequently, samples were stained with metal-conjugated antibody cocktail for intracellular proteins at 4°C for 45 minutes. Then, stained samples were washed twice in CyFACS buffer, once in PBS, and fixed overnight at 4°C in 2% formaldehyde (manufactured by Invitrogen) in PBS with Cell-ID Intercalator-103Rh (manufactured by Fluidigm).

(CyTOF Analysis)

Before data acquisition, the fixed samples were washed once with CyFACS buffer and twice with Cell Acquisition Solution (CAS) (manufactured by Fluidigm). Washed samples were suspended in CAS containing 15% EQ Four Element Calibration Beads (manufactured by Fluidigm) and filtered. Data were acquired using a Helios mass cytometer (manufactured by Fluidigm). For analysis of acquired data, live singlet cell gating and sample de-barcoding were performed using FlowJo, and subsequential analysis was performed using Cytobank ver. 9.1 (manufactured by Beckman Coulter).

The vi-SNE algorithm and FlowSOM algorithm in Cytobank were used for t-SNE 2D mapping and clustering, respectively. Heatmaps visualizing log2 fold changes of each protein expression value between each cluster and all clusters were generated using GraphPad Prism ver. 9.3.1. The protein expression value was defined as the median plus a pseudocount of 1.

(Statistical Analysis)

For statistical significance evaluation, unpaired two-sided Student's t-test was performed to compare two groups. To compare multiple groups, one-way analysis of variance with post-Tukey test was performed. Regarding statistical significance, "N.S." indicates not significant, "*" indicates p-value < 0.05, "**" indicates p-value < 0.01, and "***" indicates p-value < 0.001.
Below are the results obtained using the above materials and methods.

(Example 1) Cre/Flp-Dependent Cross-Expression of Venus and DTR in VeDTR Mice

We attempted to induce cross-expression of reporter genes (in this case, Venus) and killing genes (in this case, diphtheria toxin receptor gene (DTR)) in vivo through Cre/Flp recombinase and Flp recombinase to target specific cell subsets.
Specifically, first, as shown in Fig. 1A, we prepared a Cre/Flp dual-dependent cassette in which a dual cassette (loxP-stop-loxP and FRT-stop-FRT), containing a transcription termination sequence (tandem polyA addition signal sequence) flanked by the recognition sequences of each recombinase, was positioned upstream of DTR-IRES-Venus. Using CRISPR/Cas9 genome editing, we created mice with this cassette inserted into an intron of the Rosa26 gene locus.
As a result, we obtained two independent mouse lines (VeDTR #1 and #2). We then evaluated Venus cross-expression in tail fibroblasts prepared from them. As a result, Venus expression was not detected in VeDTR #1 cells and VeDTR #2 cells with overexpression of either Cre or Flp alone. In contrast, when both Cre and Flp were simultaneously overexpressed, Venus expression induction was detected in VeDTR #1 and VeDTR #2 cells.
Next, we examined whether VeDTR cells would show sensitivity to diphtheria toxin (DT) treatment. As a result, 72 hours after DT treatment, both VeDTR #1 cells and VeDTR #2 cells were completely eliminated by transfection of both Cre and Flp. In contrast, both lines of VeDTR cells did not show sensitivity to DT treatment when either Cre or Flp was expressed. Thus, we successfully created mouse lines (VeDTR mouse lines) that could cross-express Venus and DTR Cre/Flp dual recombinase-dependently in vitro.

(Example 2) Th1-Treg Cells Express Venus and DTR in Foxp3-Cre/Tbx21-Flp/VeDTR (LF) Mice

To attempt cross-expression of Venus and DTR in Th1-Treg cells, as shown in Fig. 1B, we prepared a cassette in which Flp fused with a 2A peptide tag was positioned at the C-terminus of the T-bet protein. Note that this Flp was codon-optimized for mammalian cells. Using CRISPR/Cas9 genome editing, we created Tbx21-Flp mouse lines with this cassette inserted into the Tbx21 gene locus.
Next, to evaluate the recombination efficiency of Flp at the Tbx21 gene locus, we created Tbx21-Flp/VeDTR (ΔloxP) mice and tested Venus expression in various types of splenocytes. As a result, moderate or high expression of Venus was detected in T cells or natural killer (NK) cells in the spleen of Tbx21-Flp/VeDTR (ΔloxP) mice. In contrast, Venus expression was lowest and almost non-existent in B cells and macrophages, showing that Flp expression and its recombination efficiency in Tbx21-Flp mice occurred highly and specifically.
Next, as shown in Fig. 2, we created triple transgenic mice derived from VeDTR (LF), Tbx21-Flp, and the Treg cell-specific Cre line Foxp3-Cre. We then analyzed Venus expression in splenocytes from Foxp3-Cre/Tbx21-Flp/VeDTR (LF) mice. As a result, Venus was detected only in CD4$^+$ T cells and not in other CD45$^+$ cells. Furthermore, using CD25 as a marker of splenic Treg cells, we analyzed in detail the expression of Foxp3 and T-bet proteins in Venus$^+$CD4$^+$ T cells. As a result, we found that almost all Venus$^+$CD25$^+$CD4$^+$ T cells expressed both Foxp3 protein and T-bet protein. Additionally, Venus$^+$CD25$^-$CD4$^+$ T cells expressed T-bet protein in a single mode. In particular, the pattern of Foxp3 protein expression in Venus$^+$CD25$^-$CD4$^+$ T cells was bimodal, suggesting that Venus$^+$CD4$^+$CD25$^-$ cells in the spleen were divided into high and low expression groups of Foxp3. In contrast, Venus-CD25$^+$CD4$^+$ T cells expressed only Foxp3 and did not express T-bet protein. Furthermore, Venus$^-$CD25$^-$CD4$^+$ T cells did not express both Foxp3 and T-bet at all, revealing that the cross-strategy using Foxp3-Cre/Tbx21-Flp/VeDTR (LF) mice could specifically label CD4$^+$ T cells expressing Foxp3 and T-bet in vivo.
Next, we examined Venus expression in CD4$^+$ T cells in vitro. Specifically, as shown in Fig. 3, Venus-naive CD4$^+$ T cells were sorted by flow cytometry and differentiated into Th1 cells, Treg cells, and Th1-Treg cells in vitro. When Foxp3 and T-bet protein expression was evaluated by flow cytometry, IFN-γ and IL-27 treatment respectively strongly induced T-bet expression in Foxp3$^+$ cells, showing IFN-γ/IL-27-dependent conversion from Treg cells to Th1-Treg cells.

Next, we evaluated whether Venus⁻ naive CD4⁺ T cells derived from Foxp3-Cre/Tbx21-Flp/VeDTR (LF) mice would show Th1-Treg cell-specific Venus expression. As a result, as shown in Fig. 4, Venus expression was observed only when naive CD4⁺ T cells differentiated into Th1-Treg cells in Foxp3-Cre/Tbx21-Flp/VeDTR (LF) mice. In contrast, in these mice, Th0, Th1, or Treg differentiation-inducing conditions did not induce Venus expression at all, revealing that Foxp3-Cre/Tbx21-Flp-dependent cross Venus expression in Th1-Treg cells was strictly controlled.

Next, we compared the effectiveness of Th1-Treg cell generation between Th1 cells and Treg cells. As a result, consistent with previous reports (Kachler, K., Holzinger, C., Trufa, D.I., Sirbu, H., and Finotto, S. (2018). Oncoimmunology 7, e1456612), TGF-β stimulated Th1-Treg cell differentiation from Th1 cells. However, the proportion of Venus⁺ cells in TGF-β-stimulated Th1 cells was significantly lower than that in IFN-γ-stimulated or IL-27-stimulated Treg cells. These findings suggested that Th1-Treg cells mainly differentiate from Treg cells but not from Th1 cells in vitro.

Next, we examined whether naturally occurring Treg cells could also differentiate into Th1-Treg cells. Since Venus⁻CD25⁺CD4⁺ T cells expressed Foxp3 protein, we isolated splenic Venus⁻CD25⁺CD4⁺ T cells as non-Th1-Treg cells in Foxp3-Cre/Tbx21-Flp/VeDTR (LF) mice and stimulated them in the presence of IFN-γ and/or IL-27. As a result, we found that over 50% of splenic Treg cells became Venus positive through treatment with IFN-γ and/or IL-27.

Furthermore, we also demonstrated that administration of DT to Foxp3-Cre/Tbx21-Flp/VeDTR (LF) mice caused depletion of Venus⁺ cells in the spleen.

Therefore, from the above data, it was demonstrated that Th1-Treg cells express Venus and can be inductively removed by DT administration in Foxp3-Cre/Tbx21-Flp/VeDTR (LF) mice.

(Example 3) Resting Th1-Treg Cells Express Genes Showing Highly Suppressive Function

To characterize the differences between Th1-Treg cells and non-Th1-Treg cells, we analyzed gene expression patterns between Venus⁺CD4⁺CD25⁺ T cells (Venus⁺Treg cells) and Venus⁻CD4⁺CD25⁺ T cells (Venus⁻Treg cells) in the spleen of Foxp3-Cre/Tbx21-Flp/VeDTR (LF) mice.

As a result, RNA-seq analysis revealed hundreds of genes were differentially expressed between Venus⁺Treg cells and Venus⁻Treg cells. More specifically, in Venus⁺Treg cells, significant upregulation of 389 genes and downregulation of 180 genes were observed (adjusted FDR < 0.05 and |log2FC| > 1). Additionally, the pattern of transcription factors and surface proteins expressed by Venus⁺Treg cells included Tbx21 and Cxcr3, overlapping with the pattern of Th1 effector cells.

Regarding Th cell lineage-specific transcription factors, it became clear that Venus⁺Treg cells had much higher expression of Tbx21, a Th1 cell lineage-specific marker, than Venus⁻Treg cells. Additionally, although not as much as Tbx21, Bcl6, a Tfh cell lineage-specific transcription factor, showed slightly but significantly higher expression in Venus⁺Treg cells compared to Venus⁻Treg cells. On the other hand, lineage-specific transcription factors such as Gata3 (Th2), Rora and Rorg (Th17), Foxp3 (Treg), and Irf4 (Th2 and Th17) were similarly expressed between Venus⁺Treg cells and Venus⁻Treg cells.

Regarding surface receptors, Venus⁺Treg cells and Venus⁻Treg cells expressed CD25 (Il2ra), Ctla4, and GITR (Tnfrsf18) at similar levels. On the other hand, Venus⁺Treg cells expressed more mRNA encoding co-inhibitory molecules such as PD-1 (Pdcd1), TIGIT (Tigit), and lymphocyte activation gene 3 (Lag3) than Venus⁻Treg cells, suggesting that Venus⁺Treg cells are better equipped to mediate suppression than Venus⁻Treg cells.

Furthermore, higher expression of PD-1 and TIGIT on Venus⁺Treg cells compared to Venus⁻Treg cells was detected by flow cytometry. Additionally, in Venus⁺Treg cells, greater amounts of LIRLB4A, CXCR5, EpCAM, ITGB1, and CD200R1 expression were observed, similar to CXCR3. Furthermore, Venus⁺Treg cells expressed more mRNA encoding Treg cell effector molecules such as IL-10 and Fgl2 than Venus⁻Treg cells. Consistent with this high IL-10 expression, mRNA encoding Prdm1 (also known as Blimp-1), a transcription factor that trans-activates IL-10 expression, was highly expressed in Venus⁺Treg cells, suggesting that unstimulated Th1-Treg cells express a gene profile showing highly suppressive ability.

To directly examine the functional differences between Venus⁺Treg cells and Venus⁻Treg cells, we compared their suppressive ability against CD4⁺ conventional T cell proliferation in vitro. As a result, as shown in Fig. 5, Venus⁺Treg cells suppressed the proliferation of CD4⁺ conventional T cells induced by α-CD3/α-CD28 stimulation more strongly than Venus⁻Treg cells. This indicates that the suppressive ability of Th1-Treg cells is higher than that of non-Th1-Treg cells.

(Example 4) Activated Th1-Treg Cells Highly Express Perforin and Antioxidant Proteins

T cell receptor (TCR) stimulation is known to strongly upregulate Treg cell suppressor function (Zhu, J., and Shevach, E.M. (2014). Nat Immunol 15, 1002-1003). To characterize TCR-stimulated Th1-Treg cells, we compared gene expression between Venus⁺Treg cells and Venus⁻Treg cells under unstimulated or α-CD3/α-CD28 stimulated conditions by RNA sequence profiling.

As a result, Venus⁺Treg cells showed overexpression of 74 genes and reduced expression of 165 genes compared to Venus⁻Treg cells. When comparing unstimulated and TCR-stimulated Venus⁺Treg cells, TCR-stimulated Venus⁺Treg cells showed overexpression of 1043 genes and reduced expression of 3303 genes compared to unstimulated cells. As a result, 40 overlapping upregulated genes were identified as signature genes of TCR-stimulated Th1-Treg cells.

Among them, as shown in Fig. 6A, Prf1 was highly expressed in α-CD3/α-CD28-stimulated Venus⁺Treg cells compared to unstimulated Venus⁻Treg cells and α-CD3/α-CD28-stimulated Venus⁻Treg cells, suggesting perforin expression in Treg cells, which is necessary for the suppression of anti-tumor immunity and is a unique characteristic of TCR-stimulated Th1-Treg cells.

Furthermore, as shown in Fig. 6B, genes involved in antioxidative stress such as Srxn1, Mt1, Mt3 were included in TCR-stimulated Th1-Treg cell signature genes, suggesting that TCR-stimulated Th1-Treg cells may be equipped to survive under oxidative stress.

To test this possibility, we compared survival rates under oxidative stress reagents such as hydrogen peroxide and tert-butyl hydroperoxide (TBHP) between Venus⁺Treg cells and Venus⁻Treg cells stimulated with α-CD3/α-CD28. As a result, as shown in Fig. 7A, TCR-stimulated Venus⁺Treg cells showed significantly higher survival rates than Venus⁻Treg cells under these oxidative conditions.

Additionally, mRNA encoding metallothionein 3 (MT3) was most differentially expressed between TCR-stimulated Venus⁺Treg cells and Venus⁻Treg cells among these antioxidant proteins. Therefore next, we evaluated the role of MT3 in Treg cell survival under oxidative stress. MT3-deficient mice were created by CRISPR/Cas9 genome editing. MT3-deficient mice were born at Mendelian ratios and were viable and fertile. T cell cellularity including Treg cells was normal in the spleen of MT3-deficient mice. Furthermore, in vitro Th1-Treg cell differentiation was comparable between wild-type and MT3-deficient Treg cells. In contrast, as shown in Fig. 7B, MT3-deficient Treg cells showed significantly lower survival rates than wild-type Treg cells under oxidative stress. From these results, we were able to demonstrate that TCR-stimulated Th1-Treg cells uniquely express a gene profile showing high resistance under oxidative stress.

(Example 5) Venus⁺CD4⁺ T Cells Are Highly Accumulated in Tumor-Bearing Foxp3-Cre/Tbx21-Flp/VeDTR (LF) Mice

We examined the localization of Th1-Treg cells in Foxp3-Cre/Tbx21-Flp/VeDTR (LF) mice. As a result, gene set enrichment analysis (GSEA) showed that gene signatures of tumor-infiltrating Treg cells from mouse melanoma and colon carcinoma, and human melanoma, colon, lung, and ovarian cancers were highly enriched in Venus⁺Treg cells compared to Venus⁻Treg cells.

Therefore next, we examined whether Venus⁺ cells were localized in mouse tumor tissues. As a result, in Foxp3-Cre/Tbx21-Flp/VeDTR (LF) mice subcutaneously injected with B16F10 melanoma cells or MC-38 colon cancer cells, we found that 40% or more of tumor-infiltrating CD4⁺ T cells expressed Venus. Furthermore, the majority of Venus⁺ tumor-infiltrating CD4⁺ T cells expressed both Foxp3 and T-bet proteins in a single mode. In contrast, such expression patterns were not observed in Venus⁻ cells, suggesting that tumor-infiltrating Venus⁺CD4⁺ T cells were Th1-Treg cells.

When comparing the proportion of Venus⁺ cells among CD4⁺ T cells in various tissues, as shown in Fig. 8, the proportion of Venus⁺ cells in both tumor tissues derived from B16F10 melanoma cells and MC-38 colon cancer cells was notably higher than in other tissues (spleen, inguinal lymph nodes, liver, lung, small intestine and large intestine, etc.). From these results, it was clearly demonstrated that high accumulation of Venus⁺ cells in tumor tissues could be detected in Foxp3-Cre/Tbx21-Flp/VeDTR (LF) mice.

(Example 6) MT3 is Essential for Accumulation of Th1-Treg Cells in Tumors

It is well known that reactive oxygen species are generated by cancer cells in tumor tissues (Aggarwal et al., 2019; Nishikawa, 2008; Szatrowski and Nathan, 1991). As described above, having found that MT3 is an important signature for TCR-stimulated Th1-Treg cells to survive under oxidative stress, we evaluated the role of MT3 in tumor infiltration of Th1-Treg cells.

As a result, as shown in Fig. 9, the proportions of tumor-infiltrating Tbet⁻Foxp3⁺CD4⁺ T cells (non-Th1-Treg cells) and T-bet⁺Foxp3⁻CD4⁺ T cells (Th1 cells) were equivalent between B16F10 tumor-bearing wild-type mice and B16F10 tumor-bearing MT3-deficient mice. In contrast, in B16F10 tumor-bearing MT3-deficient mice, the proportion of Foxp3⁺T-bet⁺CD4⁺ T cells (Th1-Treg cells) was significantly lower than in wild-type mice, indicating that MT3 plays an essential role in tumor infiltration of Th1-Treg cells.

(Example 7) Induction of Th1-Treg Cell Depletion Delays Tumor Growth

To evaluate the role of Th1-Treg cells in tumor growth in vivo, we attempted to remove (deplete) Venus⁺ cells by administering DT to tumor-bearing Foxp3-Cre/Tbx21-Flp/VeDTR (LF) mice.

As a result, as shown in Fig. 10, in both Foxp3-Cre/Tbx21-Flp/VeDTR (LF) mice bearing B16F10 melanoma or MC-38 colon carcinoma, the proportion of Venus$^+$CD4$^+$ T cells decreased by approximately 80% after DT administration compared to saline administration.

Furthermore, when measuring tumor volume over time, as shown in Fig. 11A, the size of B16F10 melanoma and MC-38 colon carcinoma tumors in DT-administered Foxp3-Cre/Tbx21-Flp/VeDTR (LF) mice was significantly smaller than tumors in saline-administered mice, demonstrating that tumor growth was suppressed by inducible Th1-Treg cell depletion.

(Example 8) Th1-Treg Cell Depletion Enhances Anti-tumor Immunity

Next, we evaluated whether Th1-Treg cell depletion would lead to activation of tumor-infiltrating immune cells. Specifically, first, CD4$^+$ T cells and CD8$^+$ T cells were isolated from B16F10 tumor tissue of Foxp3-Cre/Tbx21-Flp/VeDTR (LF) mice administered DT or saline. Then, surface expression levels of CD69 and PD-1 were measured by flow cytometry.

As a result, as shown in Fig. 11B, tumor-infiltrating Venus-CD4$^+$ T cells isolated from DT-administered Foxp3-Cre/Tbx21-Flp/VeDTR (LF) mice showed significantly higher proportions of CD69 and PD-1 than those from saline-administered mice. In contrast, the proportions of these surface markers on CD8$^+$ T cells were comparable between DT-administered and saline-administered mice, suggesting activation of intratumoral CD4$^+$ T cells due to Th1-Treg cell depletion.

Next, to evaluate changes induced by Th1-Treg cell depletion in multiple immune cell populations in tumor tissue of Foxp3-Cre/Tbx21-Flp/VeDTR (LF) mice more globally and impartially, we performed analysis using time-of-flight (CyTOF) cytometry.

By CyTOF analysis, intratumoral CD45$^+$ cells from DT or saline-administered Foxp3-Cre/Tbx21-Flp/VeDTR (LF) mice were computationally divided into 24 clusters, revealing two Treg cell clusters (T-bet$^+$ Treg and T-bet$^-$ Treg) that could be distinguished by exclusive expression of T-bet. Additionally, high DTR expression was primarily detected in T-bet$^+$ Treg cells.

Furthermore, when comparing immune cell clusters between DT administration and saline administration conditions, as shown in Fig. 12A, a dramatic decrease in the proportion of T-bet$^+$ Treg cells was observed after DT administration. In contrast, the proportion of T-bet$^-$ Treg cells remained unchanged between the two groups, clearly demonstrating specific depletion of Th1-Treg cells by DT administration in tumor-infiltrating immune cells of Foxp3-Cre/Tbx21-Flp/VeDTR (LF) mice.

Next, when exploring the remaining clusters other than Treg cells, as shown in Fig. 12B, we found that the proportions of CD69highCD4$^+$ T cells, CD69highNK cells, and NK1.1$^+$$\gamma\delta$T cells were significantly increased after DT administration. Meanwhile, the proportions of remaining lymphoid and myeloid cell subpopulations were unaffected by DT treatment.

Next, to evaluate the contribution of CD4$^+$ T cells, NK cells, and NK1.1$^+$$\gamma\delta$T cells to anti-tumor immunity, we depleted these cells in DT-treated Foxp3-Cre/Tbx21-Flp/VeDTR (LF) mice using anti-CD4 antibody and anti-NK1.1 antibody. However, although not shown in the figure, removal of NK cells or NK1.1$^+$ cells had no effect on tumor growth in the aforementioned DT-treated mice.

In contrast, as shown in Fig. 13, when all CD4$^+$ T cells, NK cells, and NK1.1$^+$$\gamma\delta$T cells were depleted, the suppression of tumor growth by DT treatment was completely reversed, and tumors showed growth similar to PBS-treated mice. This suggested that in DT-treated tumor-bearing Foxp3-Cre/Tbx21-Flp/VeDTR (LF) mice, CD4$^+$ cells and NK1.1$^+$ cells complementarily suppress tumor growth. In other words, it was suggested that anti-tumor immunity due to Th1-Treg cell depletion is complementarily mediated by CD4$^+$ cells and NK1.1-positive cells.

Thus, through high-dimensional analysis, we were able to demonstrate that Th1-Treg cell depletion enhances anti-tumor Th1 immune responses by increasing the proportions of tumor-infiltrating CD69highCD4$^+$ T cells, CD69highNK cells, and NK1.1$^+$$\gamma\delta$T cells.

(Example 9) Induction of Th1-Treg Cell Depletion Does Not Cause Autoimmunity but Shows Anti-tumor Effects Comparable to Total Treg Cell Depletion

Abundant Treg cell infiltration in tumor tissue is often associated with poor prognosis in cancer patients (Tanaka, A., and Sakaguchi, S. (2019). Eur J Immunol 49, 1140-1146). Induction of Treg cell removal not only enhances anti-tumor immunity in adult mice (Klages, K., Mayer, C.T., Lahl, K., Loddenkemper, C., Teng, M.W., Ngiow, S.F., Smyth, M.J., Hamann, A., Huehn, J., and Sparwasser, T. (2010). Cancer Res 70, 7788-7799; Mattarollo, S.R., Steegh, K., Li, M., Duret, H., Foong Ngiow, S., and Smyth, M.J. (2013). Immunol Cell Biol 91, 105-114; Teng, M.W., Ngiow, S.F., von Scheidt, B., McLaughlin, N., Sparwasser, T., and Smyth, M.J. (2010). Cancer Res 70, 7800-7809) but also triggers lethal systemic autoimmunity (Kim, J.M., Rasmussen, J.P., and Rudensky, A.Y. (2007). Nat Immunol 8, 191-197). Therefore, targeting all Treg cells has been postponed in cancer immunotherapy.

As described above, it became clear that anti-tumor immunity is enhanced by inducing Th1-Treg cell depletion.

However, it was unclear whether removal of Th1-Treg cells would lead to autoimmunity. To clarify this point, we administered DT daily to Foxp3-Cre/Tbx21-Flp/VeDTR (LF) mice, which can globally remove Treg cells upon DT administration, and control VeDTR (LF) mice.

As a result, as shown in Figure 14A, Foxp3-Cre/VeDTR (ΔFRT) mice continuously lost weight and were in a moribund state by day 10 after DT administration. In contrast, Foxp3-Cre/Tbx21-Flp/VeDTR (LF) mice and VeDTR (LF) mice maintained their health and did not lose any weight.

Additionally, as shown in Fig. 14B, while serum concentrations of aspartate aminotransferase (AST) and alanine aminotransferase (ALT) remained unchanged in DT-administered Foxp3-Cre/Tbx21-Flp/VeDTR (LF) mice and VeDTR (LF) mice, serum levels in Foxp3-Cre/VeDTR (ΔFRT) mice increased robustly.

Furthermore, DT-administered Foxp3-Cre/VeDTR (ΔFRT) mice showed severe tissue pathology manifested by massive immune cell infiltration in hepatic sinusoid-like capillaries and lungs. In contrast, DT-administered Foxp3-Cre/Tbx21-Flp/VeDTR (LF) mice and VeDTR (LF) mice showed no such pathological changes.

Next, we compared CD4$^+$ T cell activation among DT-administered Foxp3-Cre/Tbx21-Flp/VeDTR (LF) mice, Foxp3-Cre/VeDTR (ΔFRT) mice, and VeDTR (LF) mice. As a result, as shown in Fig. 15A, Venus$^-$CD4$^+$ T cells in the spleen of DT-administered Foxp3-Cre/VeDTR (ΔFRT) mice showed higher CD69 expression and lower CD62L expression, which indicate an activated state, compared to Venus$^-$CD4$^+$ T cells from DT-administered Foxp3-Cre/Tbx21-Flp/VeDTR (LF) mice and VeDTR (LF) mice. Notably, when DT was administered for 20 days, Foxp3-Cre/Tbx21-Flp/VeDTR (LF) mice showed no weight loss or increase in serum AST/ALT levels.

Next, we compared anti-tumor effects between Th1-Treg cell depletion and total Treg cell depletion. As a result, as shown in Fig. 15B, tumor growth in saline-administered Foxp3-Cre/Tbx21-Flp/VeDTR (LF) mice was comparable to tumor growth in saline-administered Foxp3-Cre/VeDTR (ΔFRT) mice. In particular, the effect of DT administration in Foxp3-Cre/Tbx21-Flp/VeDTR (LF) mice was similar to the effect in Foxp3-Cre/VeDTR (ΔFRT) mice.

From these results, it was demonstrated that induction of Th1-Treg cell depletion does not cause lethal autoimmunity but maintains anti-tumor effects comparable to total Treg cell depletion.

[Industrial Applicability]

As explained above, according to the present invention, it becomes possible to provide non-human organisms, such as Foxp3-Cre/Tbx21-Flp/VeDTR mice, that can express killing genes in response to the expression of multiple types of genes characteristic of specific cells. Furthermore, in such organisms, it becomes possible to specifically remove the cells, and by utilizing the expression of these genes, it also becomes possible to analyze gene expression, functions, etc. of the cells. This invention is useful not only in basic research but also in application fields such as pharmaceutical development.

**Claims**

1. A combination of DNA constructs for killing specific cells, comprising:

   a first DNA construct containing a first site-specific recombinase gene operably positioned relative to a promoter of a first gene characteristic of the cells, up to an n-th DNA construct containing an n-th site-specific recombinase gene operably positioned relative to a promoter of an n-th gene characteristic of the cells; and
   an (n+1)th DNA construct containing a killing gene operably positioned relative to a constitutive promoter, wherein a transcription termination sequence flanked by a pair of first recognition sequences recognized and excised by a first site-specific recombinase, up to a transcription termination sequence flanked by a pair of n-th recognition sequences recognized and excised by an n-th site-specific recombinase, are positioned between the constitutive promoter and the killing gene (where n represents a natural number of 2 or greater).

2. The combination of DNA constructs according to Claim 1, wherein at least one of the first to n-th site-specific recombinases is an enzyme that is activated in response to stimulation.

3. A cell into which the combination of DNA constructs according to Claim 1 or 2 has been introduced.

4. A non-human organism comprising the cell according to Claim 3.

5. A method for analyzing a function of specific cells, comprising the steps of:

   providing stimulation to the non-human organism according to Claim 4 to induce cell killing by the killing gene; and

comparing a phenotype of the non-human organism according to Claim 4 in which the cell according to Claim 3 has been killed and removed by the stimulation, with a phenotype of the non-human organism according to Claim 4 that has not been given the stimulation and in which the cell killing has not been induced.

6. A DNA construct for killing specific cells, comprising:

a killing gene operably positioned relative to a constitutive promoter, wherein
a transcription termination sequence flanked by a pair of first recognition sequences recognized and excised by a first site-specific recombinase, up to a transcription termination sequence flanked by a pair of n-th recognition sequences recognized and excised by an n-th site-specific recombinase, are positioned between the constitutive promoter and the killing gene (where the first to n-th site-specific recombinase genes are each expressed under the control of promoters of first to n-th genes characteristic of the cells. n represents a natural number of 2 or greater).

7. The DNA construct according to Claim 6, wherein at least one of the first to n-th site-specific recombinases is an enzyme that is activated in response to stimulation.

8. A cell into which the DNA construct according to Claim 6 or 7 has been introduced.

9. A non-human organism comprising the cell according to Claim 8.

10. A pharmaceutical composition containing the cell according to Claim 3 or 8.

Fig. 1

Fig. 2

**Fig. 3**

**(C)**

DAY    0-3 (DIFFERENTIATION INDUCTION)    3-6 (REST)    6-9 (REDIFFERENTIATION INDUCTION)

Naïve CD4

CD4$^+$
CD25$^-$
CD44$^{low}$
CD62L$^{hi}$
Venus$^-$

→ Th0

$+\alpha$-IFN-$\gamma$
$\alpha$-IL-4

→ Th1 → → Th1-Treg

**(D)**

$+$mIL-12
$\alpha$-IL-4

$+$mIL-12
$\alpha$-IL-4

$+$mIL-12
hTGF-$\beta$

→ Treg → → Th1-Treg

$+$hTGF-$\beta$
$\alpha$-IFN-$\gamma$
$\alpha$-IL-4

$+$hTGF-$\beta$
$\alpha$-IFN-$\gamma$
$\alpha$-IL-4

$+$hTGF-$\beta$
mIFN-$\gamma$ / mIL-27

| | 0-3 | 3-6 | 6-9 |
|---|---|---|---|
| $\alpha$-CD3 / $\alpha$-CD28 | + | − | + |
| mIL-2 | + | + | + |

**(E)**

Treg (Venus$^-$)

CD4$^+$
CD25$^+$
Venus$^-$

3 DAYS → Th1-Treg (Venus$^+$)

$+$
$\alpha$-CD3 / $\alpha$-CD28
IL-2
TGF-$\beta$
IFN-$\gamma$ / IL-27

Fig. 4

EP 4 578 947 A1

Fig. 5

Fig. 6

EP 4 578 947 A1

Fig. 7

Fig. 8

Fig. 9

Fig. 10

Fig. 11

Fig. 12

Fig. 13

Fig. 14

Fig. 15

# INTERNATIONAL SEARCH REPORT

| International application No. |
| --- |
| **PCT/JP2023/025274** |

**A. CLASSIFICATION OF SUBJECT MATTER**

*C12N 15/11*(2006.01)i; *A01K 67/027*(2006.01)i; *A61K 35/12*(2015.01)i; *A61K 48/00*(2006.01)i; *A61P 43/00*(2006.01)i; *C12N 1/15*(2006.01)i; *C12N 1/19*(2006.01)i; *C12N 1/21*(2006.01)i; *C12N 5/10*(2006.01)i; *C12N 15/52*(2006.01)i

FI: C12N15/11 Z; C12N15/52 Z; C12N1/15; C12N1/19; C12N1/21; C12N5/10; A01K67/027; A61K35/12; A61P43/00 105; A61K48/00

According to International Patent Classification (IPC) or to both national classification and IPC

**B. FIELDS SEARCHED**

Minimum documentation searched (classification system followed by classification symbols)

C12N15/11; A01K67/027; A61K35/12; A61K48/00; A61P43/00; C12N1/15; C12N1/19; C12N1/21; C12N5/10; C12N15/52

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | WANG, H. et al., Dual Cre and Dre recombinases mediate synchronized lineage tracing and cell subset ablation in vivo, Journal of Biological Chemistry, 2022, 298(6), No.101965, pp.1-13, Published Papers in Press, April 21, 2022<br>entirety in particular, abstract, p. 2, left column, p. 5, fig. 3, p. 6, fig. 4 | 1-10 |
| Y | | 1-10 |
| Y | GOLDBERG, M. F. and JENKINS, M. K. CD4+ T cell control of Salmonella infection and persistence is dependent on different macrophage subsets. The Journal of Immunology, 2017, vol. 198, no.1, supplement, [online], [retrieved on 01 September 2023], Internet: <URL: https://journals.aai.org/jimmunol/article/198/1_Supplement/57.7/77031/CD4-T-cell-control-of-Salmonella-infection-and><br>abstract | 1-10 |
| A | GE, M.-H. et al. Dual recombining-out system for spatiotemporal gene expression in C. elegans, iScience, 2020, vol. 23, no. 101567, pp. 1-9, Supplemental Information pp. 1-14<br>entirety in particular, abstract, p. 3, fig. 1 | 1-10 |

✓ Further documents are listed in the continuation of Box C.     ✓ See patent family annex.

| | |
| --- | --- |
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **05 September 2023** | **12 September 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| | International application No. |
|---|---|
| | **PCT/JP2023/025274** |

### C. DOCUMENTS CONSIDERED TO BE RELEVANT

| Category* | Citation of document, with indication, where appropriate, of the relevant passages | Relevant to claim No. |
|---|---|---|
| A | US 2017/0183654 A1 (TRUSTEES OF BOSTON UNIVERSITY) 29 June 2017 (2017-06-29) entirety in particular, fig. 1D, 7 | 1-10 |
| P, X | OKAMOTO, M. et al. A genetic method specifically delineates Th1-type Treg cells and their roles in tumor immunity, Cell Reports, 2023, vol. 42, no. 112813, pp. 1-26, Published: July 12, 2023 entirety in particular, abstract, fig. 1, 2 | 1-10 |

Form PCT/ISA/210 (second sheet) (January 2015)

## INTERNATIONAL SEARCH REPORT
### Information on patent family members

International application No.

**PCT/JP2023/025274**

| Patent document cited in search report | Publication date (day/month/year) | Patent family member(s) | Publication date (day/month/year) |
|---|---|---|---|
| US 2017/0183654 A1 | 29 June 2017 | WO 2015/188191 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BUCH, T. et al.** *Nat Methods*, 2005, vol. 2, 419-4
- **KOCH, M.A. et al.** *Immunity*, 2012, vol. 37, 501-510
- **KOCH, M.A. et al.** *Nat Immunol*, 2009, vol. 10, 595-602
- **CHUNG, Y. et al.** *Nat Med*, 2011, vol. 17, 983-988
- **DUHEN, T. et al.** *Blood*, 2012, vol. 119, 4430-4440
- **LINTERMAN, M.A. et al.** *Nat Med*, 2011, vol. 17, 975-982
- **ZHENG, Y. et al.** *Nature*, 2009, vol. 458, 351-356
- **KIM, J.S. et al.** *Immunity*, 2021, vol. 54, 176-190
- **MADISEN, L. et al.** *Neuron*, 2015, vol. 85, 942-958
- **HIRRLINGER, J. et al.** *PLoS One*, 2009, vol. 4, e4286
- **YU et al.** *Science*, 2009, vol. 324, 797-801
- **WING, K. et al.** *Science*, 2008, vol. 322, 271-275
- **SASAKI, I. et al.** *Blood*, 2012, vol. 120, 4733-4743
- **GE, S.X. et al.** *BMC Bioinformatics*, 2018, vol. 19, 534
- **MEI, H.E. et al.** *Cytometry A*, 2016, vol. 89, 292-300
- **STERN, A.D. et al.** *Cytometry A*, 2017, vol. 91, 14-24
- **DEVINE, R.D. et al.** *Cytometry A*, 2021, vol. 99, 1042-1053
- **KACHLER, K.** ; **HOLZINGER, C.** ; **TRUFA, D.I.** ; **SIRBU, H.** ; **FINOTTO, S.** *Oncoimmunology*, 2018, vol. 7, e1456612
- **ZHU, J.** ; **SHEVACH, E.M.** *Nat Immunol*, 2014, vol. 15, 1002-1003
- **TANAKA, A.** ; **SAKAGUCHI, S.** *Eur J Immunol*, 2019, vol. 49, 1140-1146
- **KLAGES, K.** ; **MAYER, C.T.** ; **LAHL, K.** ; **LODDEN-KEMPER, C.** ; **TENG, M.W.** ; **NGIOW, S.F.** ; **SMYTH, M.J.** ; **HAMANN, A.** ; **HUEHN, J.** ; **SPARWASSER, T.** *Cancer Res*, 2010, vol. 70, 7788-7799
- **MATTAROLLO, S.R.** ; **STEEGH, K.** ; **LI, M.** ; **DURET, H.** ; **FOONG NGIOW, S.** ; **SMYTH, M.J.** *Immunol Cell Biol*, 2013, vol. 91, 105-114
- **TENG, M.W.** ; **NGIOW, S.F.** ; **VON SCHEIDT, B.** ; **MCLAUGHLIN, N.** ; **SPARWASSER, T.** ; **SMYTH, M.J.** *Cancer Res*, 2010, vol. 70, 7800-7809
- **KIM, J.M.** ; **RASMUSSEN, J.P.** ; **RUDENSKY, A.Y.** *Nat Immunol*, 2007, vol. 8, 191-197